(19) Europäisches Patentamt
European Patent Office
Office européen des brevets

(11) EP 3 511 005 A1

(12) EUROPEAN PATENT APPLICATION

(43) Date of publication:
17.07.2019 Bulletin 2019/29

(51) Int Cl.:
*A61K 31/519* (2006.01) *A61P 43/00* (2006.01)

(21) Application number: 18151570.1

(22) Date of filing: 15.01.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(71) Applicant: Technische Universität München
80333 München (DE)

(72) Inventor: Djabali, Karima
Dietersheim (Eching) (DE)

(74) Representative: Leißler-Gerstl, Gabriele
Hoefer & Partner
Patentanwälte mbB
Pilgersheimer Strasse 20
81543 München (DE)

# (54) COMPOUNDS FOR USE IN TREATING HUTCHINSON-GILFORD PROGERIA SYNDROME

(57) Hutchinson-Gilford progeria syndrome (HGPS) is a rare premature aging genetic disorder whose genetic basis is a de novo single-base substitution within exon 11 of the lamin A gene that leads to the accumulation of progerin, a lamin A isoform. It was found that 11 out of the 14 genes that are involved simultaneously in the four main phenotypes that characterize HGPS (vascular disease, arthritis, lipodystrophy and alopecia) are regulated by the JAK-STAT pathway. By demonstrating that 10 out of these 11 genes have their expression altered in HGPS fibroblasts and that primarily STAT1 and STAT3, and to a lesser extent STAT5a, STAT5b are overexpressed in HGPS fibroblasts, indicating that the JAK-STAT pathway is altered in HGPS patients.

To try to revert this alteration, baricitinib, a JAK-STAT inhibitor, was tested on control and HGPS fibroblast cultures. It was found that baricitinib exerts beneficial effects on cellular homeostasis in HGPS through the amelioration of autophagy, proteasome and mitochondrial activity, three molecular mechanisms that are altered in HGPS cells. The inhibition of the JAK-STAT pathway by baricitinib also reduces progerin levels, proving that there is a direct connection between the JAK-STAT pathway and HGPS and that inhibition of the JAK-STAT pathway is a successful approach to treatment of HGPS.

FIG. 1

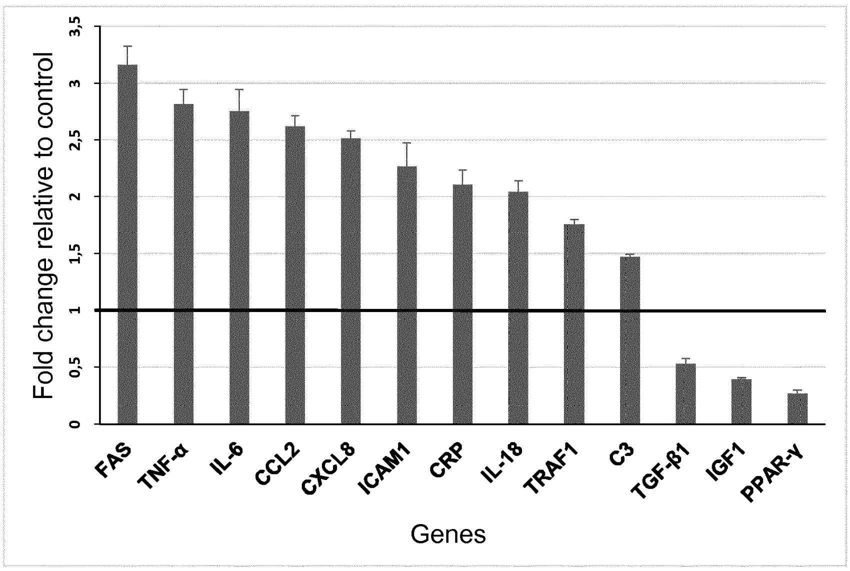


EP 3 511 005 A1

## Description

### FIELD OF THE INVENTION

The present invention is concerned with compounds, and pharmaceutical compositions comprising the compound, for use in treating symptoms of Hutchinson-Gilford progeria syndrome (HGPS) in a subject by inhibiting the JAK-STAT signaling pathway.

### BACKGROUND

Hutchinson-Gilford progeria syndrome (HGPS), or progeria, is a rare pediatric genetic disorder which was originally described in the medical literature in 1886 (J. Hutchinson) and 1897 (H. Gilford). Affected children are normal at birth and grow normally until about the end of the first year. At approximately nine to 24 months of age, affected children begin to experience profound growth delays, resulting in short stature and low weight. In addition, by the second year of life, further symptoms of HGPS will manifest. These symptoms include alopecia, which is loss of the scalp hair, eyebrows, and eyelashes, vascular disease such as generalized atherosclerosis and/or widespread arteriosclerosis, lipodystrophy, which is loss of subcutaneous adipose tissue, arthritis, and/or other abnormalities.

Approximately 200 cases have been reported worldwide. Estimates indicate that the prevalence rate of HGPS is approximately one in eight million, but if unreported or misdiagnosed cases are taken into account, the estimated birth prevalence is one in four million. According to the Progeria Research Foundation , at any given time, there are approximately 350-400 children living with progeria worldwide.

Progeria is caused by a mutation of the gene LMNA, or lamin A, a protein that is a key component of the nucleoskeleton located beneath the membrane surrounding the cell's nucleus. The abnormal lamin A protein produced in HGPS is called progerin. It is believed that the defective lamin A protein makes the nucleus unstable. That cellular instability appears to lead to the process of premature aging in progeria. Furthermore, progerin accumulation induces changes in the composition of the HGPS nuclear proteome, including alterations to several components of the protein degradation pathways. These changes result in impaired proteasome and autophagy activity in HGPS cells.

So far no effective treatment of HGPS is available. Several approaches have been attempted trying to reverse a few of the phenotypic traits that characterize HGPS. Some of the most recent and relevant examples are farnesyltransferase inhibitors (FTIs), statins, bisphosphonates, sulforaphane (SFN) and rapamycin.

FTIs block the farnesylation of prelamin A in HGPS cells. As a result, farnesylated prelamin A does not accumulate in the nuclear rim and the percentage of cells with misshapen nuclei is reduced. FTI-treated HGPS mice models exhibited improved body weight, grip strength and bone integrity, and a higher survival percentage at 20 weeks of age, suggesting that FTIs may have beneficial effects in humans with progeria. However, despite all the beneficial effects, the disease phenotypes were not completely eliminated by the FTI treatment. Furthermore, clinical trials of FTI have identified anemia, thrombocytopenia, myelosuppression, and neutropenia as most common side effects caused by the administration of FTIs

Statins are inhibitors of the cholesterol biosynthetic pathway and are widely used to reduce hypercholesterolemia and associated disorders, such as atherosclerosis. These compounds also inhibit the synthesis of isoprenoid precursors such as HMG-CoA reductase, which is involved in the protein prenylation pathway, thus reducing lamin A maturation.

Bisphosphonates, which are commonly used as therapeutic agents against disorders with increased bone resorption, reduce the synthesis of geranyl-geranyl groups and farnesyl groups by inhibiting farnesylpyrophosphate synthase. In this manner, statins and bisphosphonates have potential in the treatment of progeria syndromes or some of their symptoms, such as vascular or bone defects. In fact, combined administration of these two types of compounds has already been tested in HGPS mice models, showing that they efficiently inhibit both farnesylation and geranylgeranlyation of both progerin and prelamin A. As a result, these drugs markedly improve the aging-like phenotypes of HGPS mice, including growth retardation, loss of weight, lipodystrophy, hair loss, and bone defects, substantially extending their longevity. However, it has been found in a clinical trial that tested the therapeutic effect of the administration of a combination of a statin (pravastatin), a bisphosphonate (zoledronic acid) and a FTI (lonafarnib) in HGPS patients that no cardiovascular benefit can be observed.

Another approach attempted in the prior art is to enhance proteasome and autophagy activity in the cells comprising accumulated progerin by increasing the expression of proteasome system components and of several heats hock proteins and co-chaperone by application of SFN, a molecule found in cruciferous vegetables that has the ability to reduce oxidative stress and promote proteostasis. SFN enhances progerin clearance by autophagy and reverses some of the phenotypic changes that are the hallmarks of HGPS. Another drug that significantly reduces progerin levels in HGPS cells via autophagy is rapamycin, an inhibitor of the mTOR pathway. By inducing progerin clearance through autophagic mechanisms, rapamycin abolishes characteristic nuclear defects, markedly prolongs cellular life span, and decreases the formation of insoluble progerin aggregates.. Despite all the beneficial effects, it has been shown that

regular administration of rapamycin produces a number of side effects in humans, such as myelosuppression, hyperlipidemia and problems related to over-immunosuppression.

Although all these studies have demonstrated that FTIs, SFN, rapamycin, statins and bisphosphonates can reverse some of the characteristic phenotypic traits of HGPS, none of them can alleviate simultaneously vascular disease, arthritis, lipodystrophy and alopecia, the four main phenotypes that characterize this condition.

Therefore there is a need for a new approach for effectively treating simultaneously the main symptoms of HGPS such as vascular disease, arthritis, lipodystrophy and alopecia.

This new approach has been found and established by the applicants of the present invention.

## SUMMARY OF THE INVENTION

The present invention is concerned with a compound for use in treating Hutchinson-Gilford progeria syndrome (HGPS) in a subject suffering from HGPS. The compound is an inhibitor of the JAK-STAT signaling pathway. It has been surprisingly found that selective and reversible inhibition of this pathway leads to an amelioration of the main symptoms of HGPS, which are vascular disease, arthritis, lipodystrophy, and alopecia.

The present invention is also concerned with a pharmaceutical composition comprising a compound capable of selectively and reversely inhibiting the JAK-STAT pathway in a subject and a pharmaceutically acceptable carrier for use in treating Hutchinson-Gilford progeria syndrome in a subject.

In one embodiment the compound for use in treating Hutchinson-Gilford progeria syndrome (HGPS) in patients suffering from HGPS selectively and reversely inhibits JAK1 and/or JAK2.

In one embodiment the compound for use in treating Hutchinson-Gilford progeria syndrome (HGPS) in patients suffering from HGPS exhibits an $IC_{50}$ for JAK1 of about 6 nM or less, and wherein the compound exhibits an $IC_{50}$ for JAK2 of about 6 nM or less.

In one embodiment the compound for use in treating Hutchinson-Gilford progeria syndrome (HGPS) in patients suffering from HGPS is baricitinib having the following structure:

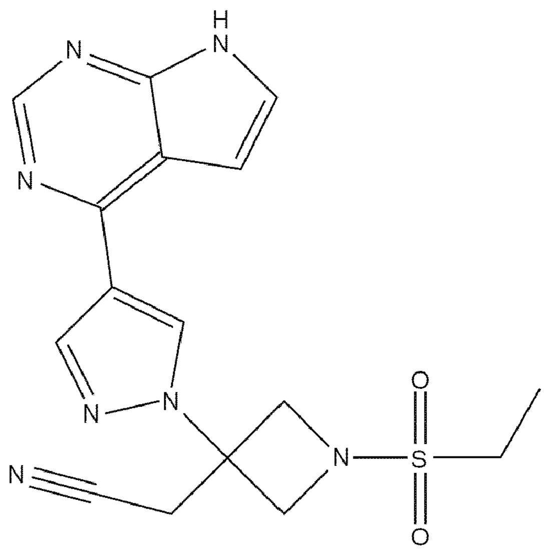

,

or a pharmaceutically acceptable salt thereof.

The present invention is therefore concerned with a compound for use in treating Hutchinson-Gilford progeria syndrome in a subject, wherein the compound selectively and reversely inhibits the JAK-STAT pathway in the subject.

## BRIEF DESCRIPTION OF THE DRAWINGS

**Figure 1** shows the relative changes in gene expression of several genes involved in the pathogenesis of the main symptoms of HGPS between HGPS fibroblasts and control fibroblasts quantified by RT-PCR. mRNA Expression levels of PPAR-γ, IGF1 and TGF-β1 were decreased, and increased for C3, TRAF1, IL-18, CRP, ICAM1, CXCL8, CCL2, IL-6, TNF-α and FAS in HGPS fibroblasts in comparison to control fibroblasts. All values are presented as the mean ± SD (*P<0.05; n=3).

**Figure 2** shows the relative changes in gene expression of several JAK-STAT pathway genes between HGPS fibroblasts and control fibroblasts quantified by RT-PCR. STAT1, STAT2, STAT3, STAT4, STAT5a, STAT5b and STAT6 encoding mRNA fold expression change in HGPS fibroblasts relative to their expression in control fibroblasts, determined by RT-PCR. All values are presented as the mean ± SD (*P<0.05; n=3).

**Figure 3** shows data obtained with a baricitinib cytotoxicity assay. The percentage of cell death obtained in a short-term (24 h) and a long-term (48 h) cytotoxicity assays performed in control cells exposed to DMSO 0.1 % v/v and baricitinib solubilized in DMSO at six different concentrations is shown: 0.125 μM, 0.25 μM, 0.5 μM, 1 μM, 2 μM, and 4 μM. All values are presented as the mean ± SD (*P<0.05; n=3).

**Figure 4** shows cumulative population doublings of control fibroblasts and HGPS fibroblasts that were fed daily with fresh medium supplemented with 0.25 μM, 0.5 μM or 1 μM baricitinib for a period of 4 days or 9 days. All values are presented as the mean ± SD (*P<0.05; n=3).

**Figure 5** shows level of autophagy relative to mock-treated control of control fibroblasts and HGPS fibroblasts that were fed daily with fresh medium supplemented with 0.25 μM, 0.5 μM or 1 μM baricitinib for a period of 4 days or 9 days. All values are presented as the mean ± SD (*P<0.05; n=3) relative to mock-treated control fibroblasts.

**Figure 6** shows proteasomal activity relative to mock-treated control of control fibroblasts and HGPS fibroblasts that were fed daily with fresh medium supplemented with 0.25 μM, 0.5 μM or 1 μM baricitinib for a period of 4 days or 9 days. All values are presented as the mean ± SD (*P<0.05; n=3) relative to mock-treated control fibroblasts.

**Figure 7** shows reactive oxygen species (ROS) levels relative to mock-treated control of control fibroblasts and HGPS fibroblasts that were fed daily with fresh medium supplemented with 0.25 μM, 0.5 μM or 1 μM baricitinib for a period of 4 days or 9 days. All values are presented as the mean ± SD (*P<0.05; n=3) relative to mock-treated control fibroblasts.

**Figure 8** shows ATP levels relative to mock-treated control of control fibroblasts and HGPS fibroblasts that were fed daily with fresh medium supplemented with 0.25 μM, 0.5 μM or 1 μM baricitinib for a period of 4 days or 9 days. All values are presented as the mean ± SD (*P<0.05; n=3) relative to mock-treated control fibroblasts.

**Figure 9** shows Western blot analysis performed on 4 days mock-treated or baricitinib-treated (at the concentrations of 0.25 μM, 0.5 μM or 1 μM) control and HGPS fibroblasts. **Panel A** shows a blot stained with Ponceau. **Panel B** shows blots probed with anti-lamin A/C, progerin and to β-action as indicated. **Panel C** shows quantification of progerin in HGPS cells treated with either the vehicle (mock) or 0.5 or 1 μM baricitinib for 4 days normalized to β-action. Levels are presented as the fold-expression relative to mock treated HGPS cells. **Panel D** shows fold-expressions of lamin A, progerin, and lamin C, which were determined for each sample and analyzed by Western blotting with anti-lamin A/C antibody in **Panel B** and normalized to β-action (*p-value ≤ 0.05).

**Figure 10** shows Western blot analysis performed on 9 days mock-treated or baricitinib-treated (at the concentrations of 0.25 μM, 0.5 μM or 1 μM) control and HGPS fibroblasts. **Panel A** shows a blot stained with Ponceau. **Panel B** shows blots probed with anti-lamin A/C, progerin and to β-action as indicated. **Panel C** shows quantification of progerin in HGPS cells treated with either the vehicle (mock) or 0.5 or 1 μM baricitinib for 9 days normalized to β-action. Levels are presented as the fold-expression relative to mock treated HGPS cells. **Panel D** shows fold-expressions of lamin A, progerin, and lamin C which were determined for each sample and analyzed by Western blotting with anti-lamin A/C antibody in **Panel B** and normalized to β-action (*p-value ≤ 0.05).

**Figure 11** shows the relative changes in gene expression of several genes involved in the pathogenesis of the main symptoms of HGPS between mock-treated HGPS fibroblasts (blue) and 9 days baricitinib-treated HGPS (orange) relative to 9-day mock-treated control fibroblasts as determined by qPCR. All values are presented as the mean ± SD.

**Figure 12** shows the difference in size between wild-type mice, heterozygous progerin mice, and homozygous progerin mice (see Osorio et al., Sci Transl. Med. 2001). It further shows the concentration of lamin A and lamin C in the heart of wildtype and homozygous mice. It is also shown that the ratios of lamin A/C and progerin in mouse homozygous heart cells (heart tissue) by comparison to human HGPS fibroblasts are very different. There is much more progerin in the homozygous progerin mouse cells.

**Figure 13** shows Western blot analysis performed on 4 and 9 days mock-treated or 1 μM baricitinib-treated control and HGPS fibroblasts. **Panel A** shows a blot stained with Ponceau. **Panel B** shows blots probed with anti-lamin A/C, progerin and β-action as indicated. **Panel C** shows quantification of progerin in HGPS cells treated with either the vehicle (mock) or 1 μM baricitinib for 4 and 9 days normalized to β-action. Levels are presented as the fold-expression relative to mock treated HGPS cells.

**Figure 14** shows data of a baricitinib cytotoxicity assay obtained in mouse fibroblasts from a progerin mouse model versus WT mouse fibroblasts. The progerin mouse is homozygous for LMNA G609G/G609G (referred to as Δ/ΔG609G/G609G mouse). To determine the non-toxic concentration of baricitinib to be used on mouse fibroblast cultures, cells were treated with different concentration of baricitinib. The results indicate that above 3 μM of baricitinib an increased number of cells in control and progerin-mouse fibroblast were dead. These data indicate that 3 μM of baricitinib should be used for treatment of mouse fibroblast cultures.

**Figure 15** shows cell growth measured in mouse fibroblast cultures treated with 3 μM of baricitinib. These results

indicate that 3 μM of baricitinib ameliorate cell growth of control and progerin mouse cells. Cells from progerin (or Δ/Δ) mouse exhibit growth retardation compared to control cells. Baricitinib increases and ameliorate the proliferation potency of the mouse progerin fibroblasts.

**Figure 16** shows autophagy levels in control and progerin mouse cells. The results indicate that 3 μM baricitinib increases the levels of autophagy in both control and progerin fibroblasts. Importantly, the levels are increased by an average of 39.9% in progerin mouse fibroblast relative to the mock-treated control cells.

Figure **17** shows the impact of baricitinib treatment on progerin levels in mouse cells. Mouse progerin cells are homozygous for the LMNA G609G/G609G signifying that they mostly produce the mutant lamin progerin and barely produce lamin A. **Panel A** shows a blot stained with Ponceau. **Panel B** corresponds to an overexposed western blot probed with anti-lamin A/C antibodies. A weak lamin A band can be visualized and high levels of progerin are observed in the mouse progerin extracts. After 4 and 9 days of 3 μM baricitinib treatment, the levels of progerin significantly decrease by 18% by day 4 and 35% by day 9. These finding indicate that baricitinib increase progerin clearance in mouse fibroblasts derived from the progerin mouse model.

## DESCRIPTION OF THE INVENTION

In order to develop a new approach for simultaneously treating the main symptoms manifested in patients suffering from HGPS, it was necessary to better understand the molecular mechanisms leading to HGPS, and particularly, the cellular and molecular mechanisms, gene connections and pathways concerned with the emergence of the main symptoms of HGPS; which are vascular disease, arthritis, lipodystrophy and alopecia.

### Vascular disease

The main cause of death in HGPS patients is accelerated cardiovascular disease with wide spread atherosclerosis that leads to myocardial infarction or stroke. Initially, children with HGPS have normal blood pressure and do not have any cardiovascular problems. However, starting from 4 years of age, murmurs (the sound of blood flowing) can be heard, and from 4 to 6 years of age patients gradually develop shortness of breath with easy fatigability and their pulse rates and blood pressure rise. During that period, HGPS patients also show signs of impaired coronary function, enlarged heart, and earlier silent infarctions. At the end phase, the children exhibit angina pectoris and extreme dyspnoea.

Progerin, the mutant lamin A expressed in HGPS cells, is widely present in the arterial walls and atherosclerotic plaques of HGPS patients, being also detected in the coronary arteries of non-HGPS aging individuals, increasing with advancing age. Possibly, this accumulation of progerin in vascular cells causes nuclear defects, premature senescence, and increased susceptibility to mechanical strain that triggers some combination of cell death and inflammatory response, resulting in atherosclerosis.

### Arthritis

HGPS patients constantly exhibit some form of osteolysis that can be found at the distal phalanges, clavicles, mandible, viscerocranium and neurocranium. In the distal phalanges, osteolysis usually starts between the ages of 1 and 2 years in the index and little fingers, gradually extending after, with both toes and fingers being affected. The characteristic narrow shoulders of HGPS patients are originated by the increasing osteolysis of the clavicles and upper ribs. Osteolysis of the mandible is marked, causing the chin to become smaller after 1 or 2 years of age. In the viscerocranium, osteolysis causes the facial bones to become smaller and thinner with age. The consequent decrease in size of the maxilla and mandible leads to crowded teeth. Due to osteolysis, fractures occur, mainly of the skull at the end stage of the disorder. The multicentric osteolyses are notable for interphalangeal joint erosions that mimic severe juvenile rheumatoid arthritis (RA), a systemic disease that leads to the progressive destruction of articular and periarticular structures. RA is characterized by the inflammation of the synovial membrane, or synovitis, which occurs when leukocytes infiltrate and accumulate in the synovium.

### Lipodystrophy

Lipodystrophy is a heterogeneous disorder characterized by selective loss of body fat and numerous metabolic complications that indicate the importance of adipose tissue as an active endocrine organ. In HGPS patients, lipodystrophy can start as early as 6 months, but may not become visible until 3 or 4 years of age. Firstly this condition becomes noticeable in the limbs, followed by the neurocranium, thorax and face. The loss of body fat and the consequent thinning of the skin make the blood vessels more visible. In the early stages of HGPS, a characteristic visible vein across the nasal bridge is one of the earliest symptoms. Other consequences of body fat loss are the appearance of prominent eyes and wrinkling and thinning of the facial skin.

## Alopecia

Usually, children with HGPS are born with normal hair texture and coloring, but between the ages of 6 months and 2 years their hair falls out. In HGPS patients, eyelashes and eyebrows also disappear; body hair in the chest, axillae, pubis or limbs is sparse or completely absent; and hair as part of secondary sexual characteristics is rare. Besides that, the remaining hair becomes lighter in color.

Hair follicle growth occurs in a four phase cycle: a long growing phase (anagen), a short transitional phase (catagen), a short resting phase (telogen), and, at the end of the resting phase, the hair falls out (exogen) and a new hair starts to grow in the follicle, beginning the cycle again. In HGPS, alopecia is a disorder of hair follicle cycling. What triggers this condition is unknown in HGPS and remains to be established. Because hair loss in HGPS patients occurs in a similar manner as in patients with alopecia areata, where infiltration of lymphocytes into the peribular space of anagen stage hair follicles and into intrafollicular locations causes an inflammation that is specific for anagen hairs, this inflammation might be occurring in HGPS as well, and might therefore be responsible for the disruption of the growing phase that causes abnormal loss of anagen hairs.

## JAK-STAT signaling pathway

It was found that one signaling pathway is involved in all of the main symptoms of HGPS. This signaling pathway is the JAK-STAT signaling pathway. The JAK-STAT signaling pathway is the main signaling mechanism for an extensive range of cytokines and growth factors, and thus it is used to transduce a multitude of signals for the development and homeostasis in humans.

This pathway transmits information received from extracellular polypeptide signals through transmembrane receptors, directly to target gene promoters in the nucleus, providing a mechanism for transcriptional regulation without second messengers. JAK activation stimulates cell proliferation, differentiation, migration and apoptosis. These processes are critical to hematopoiesis, immune development, mammary gland development and lactation, adipogenesis, sexually dimorphic growth, and other processes.

The JAK family of kinases includes JAK1, JAK2, JAK3 and TYK2. STATs comprise a family of seven structurally and functionally related latent transcription factors that reside in the cytoplasm until activated: STAT1, STAT2, STAT3, STAT4, STAT5a, STAT5b and STAT6. Signaling through the JAK-STAT pathway is initiated when a cytokine binds to its corresponding cell-surface receptor. This results in conformational changes in the cytoplasmic portion of the receptor and the subsequent activation of JAKs, which are constitutively associated with it. In turn, JAKs mediate phosphorylation at specific receptor tyrosine residues that serve as docking sites for STATs. Once recruited to the receptor, STATs become phosphorylated by JAKs on a single tyrosine residue, resulting in their activation. Activated STATs dissociate from the receptor, dimerize, translocate to the nucleus and modulate the expression of target genes.

## Identification of genes regulated by STATS via JAKS and simultaneously involved in the four main phenotypes that characterize HGPS

In order to find all the genes regulated by STATs via JAKs, a search was performed in several transcription regulation databases (see **Example 1**) After obtaining the full list of genes that are regulated by the JAK-STAT signaling pathway, a software based on scientific literature present in PubMed was developed by the inventors and used to search for the genes of that list that are simultaneously involved in the four main phenotypes that characterize HGPS: vascular disease, arthritis, lipodystrophy and alopecia (see **Example 2**).

In this search 11 genes were found: PPAR-$\gamma$, CCL2, ICAM1, FAS, HMOX1, CRP, TGF-$\beta$1, CXCL8, TNF-$\alpha$, IGF1, and IL-6. These 11 JAK-STAT regulated genes are potentially involved in the pathogenesis of HGPS and are probably what connects the JAK-STAT signaling pathway with HGPS. To further substantiate that the JAK-STAT signaling pathway plays a pivotal role in the pathogenesis of the four main phenotypes that characterize HGPS, the list of genes present in HGNC (a database of all the genes of the human genome) was also screened using the same software. In this search, besides the 11 previously found genes, 3 other genes were also found to be involved simultaneously in the four main phenotypes that characterize HGPS: C3, TRAF1 and IL-18.

The finding that 11 out of the 14 genes that are involved simultaneously in vascular disease, arthritis, lipodystrophy and alopecia are regulated by the JAK-STAT signaling pathway substantiated the fact that this pathway has an extremely important role in the pathogenesis of these four conditions. Consequently, it also substantiated the hypothesis that the JAK-STAT pathway probably has an important role in the pathogenesis of HGPS.

## Confirmation of gene expression alterations in HGPS cells compared to control cells

In a further step, this potential involvement was confirmed by assaying gene expression alterations in a HGPS

mouse and human fibroblast compared to control fibroblasts.

To confirm if the hypotheses concerning the expression level of each one of the genes in HGPS cells were right and therefore confirm if these genes are involved in the pathogenesis of HGPS, qPCR reactions were performed for each gene in control and HGPS fibroblasts. The normalized RT-PCR results are presented in **Figure 1,** a figure that shows the encoding mRNA expression fold increase or decrease for each gene in HGPS fibroblasts relative to control fibroblasts.

It was found that PPAR-$\gamma$ and IGF1 are downregulated in HGPS, and that CCL2, CXCL8, ICAM1, CRP, TRAF1, IL-18, IL-6, TNF-$\alpha$ and FAS are overexpressed in HGPS. It was also found that although TGF-$\beta$1 and C3 expression changes in HGPS, it is visible that among the downregulated genes in HGPS, TGF-$\beta$1 has the lowest fold decrease, and among the overexpressed genes in HGPS, C3 has the lowest fold increase. This is probably related to the fact that, contrary to the other genes, these two do not have the same expression tendency in all four conditions that characterize HGPS: TGF-$\beta$1 is downregulated in vascular disease and arthritis but overexpressed in lipodystrophy and alopecia, and C3 is overexpressed in all conditions except lipodystrophy in which it is downregulated.

With the qPCR experiment, it was proven that 13 out of the 14 genes that are involved simultaneously in the pathogenesis of the four main phenotypes that characterize HGPS have their expression altered in HGPS fibroblasts, confirming that these genes are involved in the pathogenesis of HGPS.

Since 10 out of these 13 genes are regulated by the JAK-STAT pathway, there is again a strong suggestion that this pathway is actively involved in the pathogenesis of HGPS. To further substantiate this hypothesis, qPCR reactions for STAT1, STAT2, STAT3, STAT4, STAT5a, STAT5b and STAT6 were performed in control and HGPS fibroblasts to determine if there's any alteration in the expression of JAK-STAT pathway genes in HGPS. The normalized RT-PCR results are presented in **Figure 2,** a figure that shows the encoding mRNA expression fold increase or decrease for each gene in HGPS fibroblasts in relation to control fibroblasts. The results show that STAT1 and STAT3 were the most significantly altered and were upregulated in HGPS cells.

From the 11 genes regulated by the JAK-STAT pathway that were found to be simultaneously involved in the pathogenesis of the four main phenotypes that characterize HGPS, 7 are regulated by STAT1, none by STAT2, 9 by STAT3, none by STAT4, 3 by STAT5a, 3 by STAT5b and 1 by STAT6. This is in accordance with the results that were obtained in this experiment. STAT3 is the STAT that regulates the highest amount of genes simultaneously involved in the pathogenesis of the four main phenotypes that characterize HGPS and so has the highest level of overexpression in HGPS, immediately followed by STAT1; STAT5a and STAT5b regulate a lower amount of genes and, accordingly, have significantly lower levels of overexpression in comparison to STAT1 and STAT3; STAT6 only regulates one gene and thus is only slightly overexpressed in HGPS fibroblasts; STAT2 and STAT4 do not regulate any of the genes and so their expression does not change between HGPS and control fibroblasts.

These results confirm that there is an alteration in the JAK-STAT signaling pathway in HGPS fibroblasts and undeniably endorse the hypothesis that there is a direct association between the JAK-STAT pathway and HGPS. The overexpression and/or activation of the JAK-STAT pathway very likely contribute to the pathogenesis of HGPS.

**Effects of JAK-STAT inhibition**

Having substantiated in two different ways that the JAK-STAT pathway is altered in HGPS and most likely actively involved in the pathogenesis of the disease, the next step in the project was to try to reverse these gene expression alterations that occur in HGPS to determine if it results in the amelioration of some of the typical phenotypic traits that characterize this condition.

A JAK-STAT inhibitor was used to try to reduce the expression of STAT1, STAT3, and normalize STAT5a, STAT5b, and possibly correct the expression levels of the 11 JAK-STAT regulated genes that are involved simultaneously in the pathogenesis of the four main phenotypes that characterize HGPS.

From all the existing JAK-STAT inhibitors, the one that was selected was baricitinib, a drug that potently inhibits JAK1 and JAK2, but has a lower potency against TYK2 and JAK3.

Baricitinib (LY3009104, formerly INCB028050) is an orally administered, potent, selective and reversible inhibitor of the JAK-STAT pathway. This drug potently inhibits JAK1 ($IC_{50}$=5.9 nM) and JAK2 ($IC_{50}$=5.7 nM), but has a lower potency against TYK2 ($IC_{50}$=53 nM) and JAK3 ($IC_{50}$=560 nM).

As the JAK-STAT signaling pathway is involved in many biological processes, it is very important to control any sort of manipulation of this pathway to avoid undesired side effects. Therefore it was necessary to assay the cytotoxicity of the compound used to treat HGPS cells. A suitable compound selectively and reversely inhibits the JAK-STAT signaling pathway. In this way manipulation of the pathway can be controlled and reversed if necessary, for example by choosing the correct dosage of a compound with a suitable clearance.

Therefore a cytotoxicity assay was performed with baricitinib to determine the suitable concentration that maintains efficacy and does not lead to cytotoxicity (see **Example 5**). The results are shown in **Figure 3.** Based on these results an upper concentration of 1 $\mu$M baricitinib was chosen for further assays for HGPS cells. For mouse fibroblasts

obtained from a progerin mouse model, an upper concentration of 3 μM baricitinib was chosen for further assays (see **Figure 14**).

The next step was to determine if the inhibition of the JAK-STAT pathway by baricitinib exerts any beneficial effects in HGPS fibroblasts and, if so, which of the tested concentrations and what treatment duration is the most beneficial to the cells. Five functional assays were performed on cells treated for 4-days and 9-days either mock-treated or baricitinib-treated (at the concentrations of 0.25 μM, 0.5 μM and 1 μM): growth rate (cumulative population doubling assay), autophagy and proteasome activities, the ROS and ATP levels (see **Examples 6 - 10,** and **Figures 4 - 8;** for data obtained with mouse fibroblasts obtained from a progerin mouse model, see **Figures 15** and **16**)

It was confirmed that autophagy and proteasome activity are impaired in HGPS cells and also that these cells exhibit mitochondrial dysfunction. These cellular processes are closely associated, as elevated ROS levels promote an increase in protein misfolding, and the decrease in ATP reduces the ability of nuclear proteasomes to degrade ubiquitinylated proteins and has a negative impact in apoptosis. The combination of all these factors stimulates protein modification and accumulation, causing cellular dysfunction and a general loss of proteostasis that contributes in part to the premature aging process.

It was further shown that the inhibition of the JAK-STAT pathway by baricitinib exerts beneficial effects on cellular homeostasis in HGPS since it leads to an increase in autophagy and proteasome activity, to a decrease in ROS levels, and to an increase in ATP levels in HGPS fibroblasts. All these beneficial effects caused by a JAK-STAT inhibitor confirm, again, that there is a strong association between the JAK-STAT pathway and HGPS and that this pathway's alteration in HGPS very likely contributes to the pathogenesis of the disease. Moreover, from all the different conditions tested, the 1 μM baricitinib treatment with the duration of 9 days is the one that produces the highest beneficial effect in HGPS fibroblasts.

Proving that the inhibition of the JAK-STAT pathway by baricitinib ameliorates autophagy, proteasome and mitochondrial activity in HGPS fibroblasts is important and it shows that baricitinib is a promising drug to be used in the therapy of HGPS patients since it can revert some of the characteristic phenotypic traits that impair the condition of HGPS cells. Baricitinib is however only one example for a compound suitable for use in treating HGPS. Any compound exhibiting similar characteristics as baricitinib can be used for treating HGPS. For example any compound that selectively and reversely inhibits JAK1 with an $IC_{50}$ of about 6 nM, and JAK2 with an $IC_{50}$ of about 6 nM is suitable for treating HGPS.

$IC_{50}$ is the half maximal inhibitory concentration and is a measure of the effectiveness of a substance in inhibiting a specific biological or biochemical function. This quantitative measure indicates how much of a particular drug or other substance (e.g. a JAK-STAT inhibitor) is needed to inhibit a given biological process (such as the processes at the root of the main symptoms in HGPS) by half. Values of $IC_{50}$ can be obtained by the person of skill in the art with assays established in the art and can be compared with the values exhibited by baricitinib in the same type of assay.

It was further shown that JAK-STAT inhibition resulted in a reduction of levels of progerin by an average of 15% after 4 days treatment with 1 μM baricitinib as determined with anti-progerin and anti-lamin A/C antibodies (see **Figure 9 A and D**). Furthermore, progerin levels were decreased by 32 to 33% by 9 days treatment with 1 μM baricitinib (see **Figure 10**). It was therefore shown that long-term baricitinib treatment significantly increased the clearance of progerin in HGPS cells while lamin A and lamin C levels remained constant. In mouse fibroblasts obtained from a progerin mouse model, it was shown that the progerin levels were decreased by 35.6 % by 9 days treatment with 3 μM baricitinib (see **Figure 17**).

Collectively, the results indicate that inhibition of JAK-STAT signaling via 1 μM baricitinib treatment ameliorates proteostasis (autophagy and proteasome activities) and consequently increase progerin clearance in HGPS cells. This reduction in progerin content by an average of 33% within a period of 9 days is higher than the reduction observed after treatment with sulforaphane and rapamycin, which reduced progerin by approximately 20 to 25% in average in a period of 9 days. Altogether, these results indicate that baricitinib treatment ameliorates the ratios of lamin A/C in HGPS and therefore might significantly improve other cellular functions that are altered by the build-up of progerin in HGPS nuclear compartment. Data obtained in mouse fibroblasts from a progerin mouse model confirm these results (wherein the baricitinib concentration used was 3 μM).

It was therefore found that a JAK-STAT pathway inhibitor could be used as a drug to ameliorate the condition of HGPS patients. Treatment of HGPS fibroblasts with JAK-STAT pathway inhibitor induced amelioration of autophagy, proteasome and mitochondrial activity that are known to malfunction in HGPS cells. These positive effects consequently reduce the levels of progerin, the mutated form of lamin A whose accumulation in HGPS nucleus is the main originator of the phenotypic traits that characterize HGPS.

As used herein, the term treating" or "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive

treatment, that is, treatment employed to supplement another specific therapy directed toward the improvement of the associated disease, pathological condition, or disorder. Specifically the term "treatment" includes treating of HGPS by ameliorating the main symptoms of HGPS, which are vascular disease, arthritis, lipodystrophy, and alopecia.

## EXAMPLES

### Example 1 - Resources used to find the list of genes regulated by the JAK-STAT signaling pathway

In order to find all the genes regulated by STATs via JAKs, a search was performed in four different transcription regulation databases: Transcriptional Regulatory Relationships Unraveled by Sentence-based Text mining (TRRUST); Transcriptional Regulatory Element Database (TRED); Encyclopedia of DNA Elements (ENCODE); and Neph2012. TRRUST is a manually curated reference database of human transcriptional regulatory interactions (Han et al., 2015); TRED is an integrated repository for both cis- and trans- regulatory elements in mammals (Jiang et al., 2007); ENCODE is a database in which putative human transcription factors interactions with DNA are named based on ChIP-seq data (2012); and Neph2012 is a regulatory network in which transcription factor targets were discovered by DNase footprinting and transcription factor recognition sequences (Neph et al., 2012). After obtaining the complete list of genes regulated by the JAK-STAT signaling pathway using these databases, the unintentional repetition data was removed manually (some genes are mentioned more than once in the list because they have different aliases in different databases). In this search 269 genes were found, out of which 119 are regulated by STAT1, 10 by STAT2, 157 by STAT3, 14 by STAT4, 32 by STAT5a, 27 by STAT5b and 43 by STAT6.

The full list of genes that were found in this search to be regulated by STAT1, STAT3, STAT5a, STAT5b and STAT6 is shown in **Table 1.** It is possible to observe that a great number of genes are simultaneously regulated by more than one STAT.

**Table 1** - List of genes regulated by the JAK-STAT pathway

| STAT1 | | STAT3 | | STAT1,3 | STAT3,5a,5b |
|---|---|---|---|---|---|
| ACAT1 | PSMB9 | AKAP12 | MDC1 | A2M | CCND1 |
| APOE | PTGFR | AKT1 | MIA2 | B3GAT3 | CCND3 |
| BAX | REV3L | BIRC5 | MICA | CCL2 | ESR2 |
| CASP4 | S100A10 | BST2 | MMP1 | CDKN1A | IGF1 |
| CCL3 | SCARB1 | CCL20 | MMP14 | CYP19A1 | OSM |
| CCR1 | STAT2 | CD46 | MMP2 | FAS | **STAT1,3,6** |
| CCR5 | STAT3 | CDH1 | MMP3 | FCGR1A | FOS |
| CD22 | TAP1 | CDK4 | MMP7 | FGF2 | IRF1 |
| CD40LG | TBX21 | CDKN1B | NANOG | GAST | OPRM1 |
| CD86 | TLR3 | CEBPB | NDUFA13 | HMOX1 | PTGS2 |
| CEBPD | TNFSF13B | CFB | NME1 | HSPB1 | SOCS1 |
| CEBPE | UPP1 | CFLAR | NOSTRIN | HSPCA | **STAT5a,5b** |
| CFTR | XAF1 | CHI3L1 | NR0B1 | ICAM1 | CEL |
| CIITA | **STAT6** | COPS5 | OAS1 | JAK2 | ESR1 |
| CLC | ADAM8 | CRP | OXTR | JAK3 | PAX5 |
| CSF1 | ADRA2B | CSRP1 | PAX3 | JUN | **STAT1,6** |
| CTSB | ALOX12 | CTGF | PGF | KRT17 | CD40 |

| | | | | | |
|---|---|---|---|---|---|
| CTSL | ALOX15 | CYR61 | PHB | MMP9 | EGR1 |
| CXCL10 | CCL17 | DDIT3 | PIAS3 | MUC4 | IL1R1 |
| CXCL9 | CCL26 | DNMT1 | POU2F1 | MYC | **STAT3,6** |
| EDN1 | CNR1 | ETV6 | PRDM1 | NOL3 | CCL11 |
| FCGR3A | COL1A1 | F2R | PTEN | NOS2A | CISH |
| FCGRT | COL1A2 | FAAH | PTPN6 | NOX5 | CXCL8 |
| FGFR3 | CYP2E1 | FASN | ROR1 | OPRM1 | **STAT1,5a,5b** |
| FOXP3 | CYSLTR1 | FGF1 | S1PR1 | REG1A | MET |
| GATA3 | DUSP1 | FGG | SAA1 | STRA13 | RNMT |
| GBP1 | EGLN3 | FGL1 | SALL4 | TIMP1 | **STAT1,3,5a,5b,6** |
| GLS | FCER2 | FLT3 | SHH | TLR2 | TNFRSF5 |
| HSP90AA1 | FOSB | GFAP | SLC9A3 | TNFRSF8 | |
| IFIT3 | HSD3B2 | HAMP | SOS1 | TP53 | |
| IFNA1 | IL13 | HGF | STS | VIP | |
| IFNB1 | IL24 | HIF1A | TERT | **STAT1,3,5a,5b** | |
| IFNLR1 | IL4 | HP | TGFB1 | BCL6 | |
| IL1B | IRF4 | HSPA4 | TNFRSF10B | EGFR | |
| IL27 | ITGA2B | HSPCB | TP63 | IFNG | |
| IRF7 | NCOA3 | IFNAR1 | TRH | IL2RA | |
| IRF8 | ORMDL3 | IKBKE | TWIST1 | IL6ST | |
| IVL | PRKCA | IL11 | TWIST2 | MUC1 | |
| LTC4S | RHOA | IL1RN | TYK2 | PBF | |
| LY96 | SELE | IL2 | UCP2 | PIM1 | |
| MAT2A | SELP | IL21 | USP7 | PRF1 | |
| MHC2TA | SIDT1 | JUNB | VDR | SEC6L1 | |
| MMP13 | STAT1 | KIAA0146 | VEGFA | TIMP3 | |
| MVP | **STAT5b** | KLF11 | VEGFB | **STAT3,5a** | |
| NOS2 | ANGPTL4 | LBP | ZFP36 | IL10 | |
| NOX1 | RARA | LCAT | **STAT5a** | BCL2 | |
| NOX4 | **STAT1,5b** | LEP | CDKN2B | CCND2 | |
| NR1H4 | PPARG | LGALS3BP | CSN2 | **STAT3,5a,5b,6** | |
| PDCD1 | **STAT1,3,5a** | LTBP1 | SUMO1 | BCL2L1 | |
| PLAU | IL6 | MCL1 | TRIP15 | TNF | |

STAT1 and STAT3 are the STATs that regulate the highest number of genes, suggesting that these two are the most important activators of transcription in the JAK-STAT pathway in humans. The genes regulated by STAT2 and STAT4 are represented in in **Table 2** and **Table 3,** respectively. The STATs that simultaneously regulate these genes with STAT2 and STAT4 are also represented in the table.

**Table 2 -** STAT2 regulated genes and other STATs that regulate the genes simultaneously with STAT2 (✓ - regulated; × - not regulated).

| | STAT1 | STAT3 | STAT4 | STAT5a | STAT5b | STAT6 |
|---|---|---|---|---|---|---|
| APOE | ✓ | × | × | × | × | × |
| IFIT3 | ✓ | × | × | × | × | × |
| IFNA1 | ✓ | × | × | × | × | × |
| IRF1 | ✓ | ✓ | ✓ | × | × | ✓ |
| IRF7 | ✓ | × | × | × | × | × |
| NOS2 | ✓ | × | × | × | × | × |
| PDCD1 | ✓ | × | × | × | × | × |
| PML | ✓ | × | × | × | × | × |
| PTGS2 | ✓ | ✓ | × | × | × | ✓ |
| SCARB2 | × | × | × | × | × | × |

**Table 3 -** STAT4 regulated genes and other STATs that regulate the genes simultaneously with STAT4 (✓ - regulated; × - not regulated).

| | STAT1 | STAT2 | STAT3 | STAT5a | STAT5b | STAT6 |
|---|---|---|---|---|---|---|
| AICDA | × | × | × | × | × | × |
| IFNG | ✓ | × | ✓ | ✓ | ✓ | × |
| IL2RA | ✓ | × | ✓ | ✓ | ✓ | × |
| IRF1 | ✓ | ✓ | ✓ | × | × | ✓ |
| MYC | ✓ | × | ✓ | × | × | × |
| NOX1 | ✓ | × | × | × | × | × |
| NOX4 | ✓ | × | × | × | × | × |
| PBF | ✓ | × | ✓ | ✓ | ✓ | × |
| PIM1 | ✓ | × | ✓ | ✓ | ✓ | × |
| PRF1 | ✓ | × | ✓ | ✓ | ✓ | × |
| S100A4 | × | × | × | × | × | × |
| SOCS3 | ✓ | × | ✓ | × | × | × |
| STAT1 | - | × | × | × | × | ✓ |
| TBX21 | ✓ | × | × | × | × | × |

**Example 2 - Gene-disorder association finding software**

After obtaining the full list of genes that are regulated by the JAK-STAT signaling pathway, a software was used to search for the genes of that list that are simultaneously involved in the four main phenotypes that characterize HGPS: vascular disease, arthritis, lipodystrophy and alopecia. This software was developed by the inventors and it is based on scientific literature present in PubMed. Besides this list of genes, all genes present in the HGNC (Human Gene Nomenclature Committee) official list were also screened.

The first step on this search was the retrieval of all papers in which these four conditions were mentioned individually or together. The PubMed database (an NCBI database that comprises more than 27 million citations for biomedical literature from MEDLINE, life science journals and online books) was used as the key source for searching, thus abstracts or articles were saved locally using the PubMed search engine. Two of the software's functions are used

to import either the text file or the XML file of each saved paper, constructing the starting point for all subsequent processing. Papers with publication types that were not related to research or reviews (for example bibliographies, comments, or editorials) were filtered out to eliminate false-positive findings.

Following paper retrieval, the software was used to search for gene-disorder associations. Although many existing tools still use simple co-occurrence to show gene-disorder associations, this is not reliable when the objective is to search for papers in which specific associations between a specific gene and a specific disorder are tested and reported. For example, sentences like "genes A, B and C were tested but only C was highly expressed in N disease" from the methods section of the abstract, or "X algorithm has successfully demonstrated that gene A is associated with M disease and now the objective is to use this software to find genes related to N disease" from the background section of the abstract can exist in a paper, but the paper might not prove that gene A is associated with the N disease. Therefore, one of the software's functions takes this into consideration, assuming that the main findings of a research paper must be reported in the results/conclusions sections of the abstract or in the title. Consequently, in each retrieved paper, the title and results/conclusions sections of the abstract were screened to identify if any of the genes present in the two lists that were analyzed were mentioned or not. At the end of the search, the number of papers in which a gene is associated with each of the diseases is exported to a table. That way it is possible to identify all the genes that are involved simultaneously in the four main phenotypes that characterize HGPS.

**Example 3 - Cell culture and baricitinib treatment**

Primary dermal fibroblasts from subjects with HGPS were obtained from the Progeria Research Foundation. Two different fibroblasts derived from HGPS patients carrying the LMNA mutation G608G were used: HGADFN003 (male, 2 years old) and HGADFN127 (female, 3 years and 9 months old). Control dermal fibroblasts were obtained from the Coriell Institute for Medical Research (Camden, NJ). The following cell lines were used: GMO1651C and GMO3349C. All cells were maintained at -80 °C in 90% fetal bovine serum (FBS) and 10% dimethyl sulfoxide (DMSO). The Institutional Review Board of the Technische Universität München (TUM) approved the use of human cells established from skin biopsies from patients with HGPS and unaffected individuals in this study.

Control and HGPS fibroblasts were cultured at 37 °C and 5% $CO_2$ in Dulbecco's Modified Eagle Media (DMEM) (Gibco by life technologies) containing 15% FBS (Gibco by life technologies), 1% L-glutamine (Gibco by life technologies), 1% penicillin/streptomycin (Gibco by life technologies), and 2 ml of gentamycin (Gibco by life technologies). Mock-treated and baricitinib-treated fibroblasts were cultured in parallel. Baricitinib (Selleckchem) was added to the media at a concentration of 0.125 μM, 0.25 μM, 0.5 μM, 1 μM, 2 μM or 4 μM. Three times a week the cells were re-fed with new culture media.

Fibroblasts cease proliferating once they become confluent (when they completely cover the surface of the cell culture vessel) and some die if left in this state for too long. In order to keep the cells healthy and actively growing, they were periodically subcultured at approximately 80% confluency. The first step of this process is the removal of the culture media, washing with PBS (Sigma), and the addition of 1 ml of a 0.1 % sterile trypsin solution (Gibco by life technologies) to dissociate adherent cells from the vessel. After trypsinization, the cells are diluted with 8 ml of new culture media, counted with a CASY® Cell Counter (Roche), subdivided into new vessels and incubated in the same conditions.

**Example 4 - Real-time PCR analysis**

RT-PCR reactions for the genes PPAR-γ, IGF-1, TFG-β1, C3, TRAF1, IL-18, CRP, ICAM1, CXCL8, CCL2, IL-6, TNF-α, FAS, STAT1, STAT2, STAT3, STAT4, STAT5a, STAT5b and STAT6 were performed to compare their expression level between control and HGPS fibroblasts. For PPAR-γ, IGF1, TFG-β1, CRP, ICAM1, CXCL8, CCL2, IL-6, TNF-α and FAS, RT-PCR reactions were also performed to compare their expression level between mock-treated and 9-day 1 μM baricitinib-treated HGPS fibroblasts.

**RNA extraction**

RNA was isolated from control and HGPS fibroblasts (mock-treated or 9-day 1 μM baricitinib-treated) using an RNeasy® Plus Mini Kit (Qiagen). Following trypsinization, the cell number was counted with a CASY® Cell Counter (Roche) and the cellular suspensions were centrifuged at 1200 rpm for 5 min to remove the culture media. After aspirating the supernatant media, 350 μl of Buffer RLT were added to the pellet to disrupt the fibroblasts. The cell lysate was then homogenized by being pipetted to into a QIAshredder spin column (a biopolymer-shredding system in a microcentrifuge spin-column format) and centrifuged at 13.000 rpm for 2 min. To precipitate the RNA, 1 volume (350 μl) of 70% ethanol (VWR Chemicals) was added to the homogenized lysate, that was then transferred to an RNeasy spin column and centrifuged for 15 s at >10.000 rpm. 700 μl of Buffer RW1 were added to the spin column and centrifuged for 15 s at >10.000 rpm in order to wash its membrane, removing biomolecules such as carbohydrates, proteins and fatty acids

that are non-specifically bound to the silica membrane (RNA molecules larger than 200 bases remain bound to the column). Next, 500 μl of buffer RPE were added to the spin column and centrifuged for 15 s at >10.000 rpm to remove traces of salts which were still in the column due to buffers used earlier in the protocol. This step was then repeated, but with a centrifugation lasting for 2 min, to ensure that all ethanol was removed. Finally, 30-50 μl of RNase-free water were added to the spin column and centrifuged for 1 min at >10.000 rpm in order to elute the RNA. The amount of RNA obtained at the end of the procedure was determined using a NanoDrop® ND-1000 Spectrophotometer.

**cDNA synthesis**

**cDNA** was synthesized from the extracted RNA samples using an Omniscript Reverse Transcription Kit (Qiagen). First, a fresh master mix containing all the components required for first-strand synthesis except the template RNA was prepared on ice and thoroughly mixed: 2 μl of 10x Buffer RT, 2 μl of dNTP Mix (5 mM each dNTP), 2 μl of 10 μM Oligo-dT primer, 1 μl of 10 units/μl RNase inhibitor and 1 μl of Omniscript Reverse Transcriptase. Then, 2000 ng of template RNA were added to the mix and RNase-free water was added until a total volume of 20 μl was reached. Lastly, the mix was incubated for 60 min at 37 °C.

**Real-time PCR**

The list of genes evaluated by RT-PCR and their respective primers (Thermo Fisher Scientific) are presented in the following **Table 4.**

**Table 4 -** Nucleotide sequence, molecular weight (MW), GC content and melting temperature (MT) of the forward (fw) and reverse (rv) primers used in the PCR reaction for the genes PPAR-γ, CCL2, TFG-β1, CXCL8, ICAM1, CRP, C3, TRAF1, IL-18, IGF1, IL-6, TNF-α, and FAS, STAT1, STAT2, STAT3, STAT4, STAT5A, STAT5B, STAT6, JAK1, JAK2, JAK3, TYK2.

| GENE | Type | Sequence (5' to 3') | MW (μg/μmol) | %GC | MT (°C) |
|---|---|---|---|---|---|
| PPAR-γ | fw | GCT TTT CAG AAA TGA CCA TGG (SEQ ID NO:1) | 6446.2 | 43 | 55.8 |
| | rv | TGA TTG CAC TTT GGT ACT CTT GA (SEQ ID NO:2) | 7036.6 | 39 | 59.8 |
| CCL2 | fw | TAG AAG AAT CAC CAG CAG CA (SEQ ID NO:3) | 6129.0 | 45 | 57.1 |
| | rv | GTG CCT CAG TTT TCC CAT T (SEQ ID NO:4) | 5721.8 | 47 | 57.6 |
| TGF-β1 | fw | CCC ACA ACG AAA TCT ATG AC (SEQ ID NO: 5) | 6040.0 | 45 | 55.5 |
| | rv | TTG CTG TAT TTC TGG TAC AGC (SEQ ID NO:6) | 6419.2 | 43 | 55.7 |
| CXCL8 | fw | GGT GCA TAA GTT CTC TAG TAG GGT (SEQ ID NO:7) | 7439.8 | 46 | 57.2 |
| | rv | TAG AAG CTT GTG TGC TCT GC (SEQ ID NO: 8) | 6140.0 | 50 | 57.0 |

(continued)

| GENE | Type | Sequence (5' to 3') | MW (μg/μmol) | %GC | MT (°C) |
|---|---|---|---|---|---|
| ICAM1 | fw | ACA GTG ACC ATC TAC AGC TTT C (SEQ ID NO:9) | 6655.4 | 45 | 56.1 |
| | rv | CCA TAC AGG ACA CGA AGC T (SEQ ID NO: 10) | 5791.8 | 53 | 56.2 |
| CRP | fw | AAG GCA AGA GAT CTA GGA CTT CTA G (SEQ ID NO:11) | 7740.0 | 44 | 58.1 |
| | rv | TGA GAG AGG CTG GTC AAG AC (SEQ ID NO:12) | 6232.0 | 55 | 58.1 |
| C3 | fw | GCG CAT TCC GAT TGA G (SEQ ID NO:13) | 4898.2 | 56 | 57.1 |
| | rv | TCA CTG CCT GAG TGC AAG (SEQ ID NO:14) | 5500.6 | 56 | 56.9 |
| TRAF1 | fw | CAG AGG GTG GTG GAG CTT (SEQ ID NO:15) | 5636.6 | 61 | 58.7 |
| | rv | AGT GTA GAA GGC TGG GGA GA (SEQ ID NO:16) | 6312.0 | 55 | 58.9 |
| IL-18 | fw | TTG TCG CAG ATG GCT CT (SEQ ID NO:17) | 5193.4 | 53 | 56.2 |
| | rv | TTA CCA TCA TCT TCA GCT ATA AAG T (SEQ ID NO:18) | 7576.0 | 32 | 55.7 |
| IGF1 | fw | GAT TAC ACC TAC AGT GAA GAT GCA (SEQ ID NO:19) | 7370.8 | 42 | 58.3 |
| | rv | CCT GTG GGC TTG TTG AAA TA (SEQ ID NO: 20) | 6164.0 | 45 | 58.6 |
| IL-6 | fw | AAG GAG AGT CAC AGG TGA GCT (SEQ ID NO:21) | 6545.2 | 52 | 58.1 |
| | rv | CAA GTC TCC TCA TTG AAT CCA G (SEQ ID NO:22) | 6655.4 | 45 | 58.8 |
| TNF-α | fw | GAG GGA AGA GTT CCC CA (SEQ ID NO:23) | 5245.4 | 59 | 55.8 |
| | rv | CTT GAG GGT TTG CTA CAA CA (SEQ ID NO: 24) | 6133.0 | 45 | 56.4 |
| FAS | fw | TCC TCC AGG TGA AAG GAA (SEQ ID NO:25) | 5533.6 | 50 | 56.6 |
| | rv | ACT TCT AAG CCA TGT CCT TCA (SEQ ID NO:26) | 6317.2 | 43 | 56.6 |
| STAT1 | fw | TTC AGA GCT CGT TTG TGG TG (SEQ ID NO: 27) | 6171.0 | 50 | 60.0 |
| | rv | AGA GGT CGT CTC GAG GTC AA (SEQ ID NO: 28) | 6183.0 | 55 | 60.0 |

(continued)

| GENE | Type | Sequence (5' to 3') | MW (μg/μmol) | %GC | MT (°C) |
|---|---|---|---|---|---|
| STAT2 | fw | CCA AGG CTA CCA TGC TAT TC (SEQ ID NO: 29) | 6038.0 | 50 | 57.3 |
| | rv | GCT GGT CTT TCA GTT GGC TG (SEQ ID NO: 30) | 6147.0 | 55 | 61.0 |
| STAT3 | fw | CAG GAG GGC AGT TTG AGT CC (SEQ ID NO:31) | 6199.0 | 60 | 62.1 |
| | rv | CAA AGA TAG CAG AAG TAG GAG A (SEQ ID NO:32) | 6875.4 | 41 | 53.4 |
| STAT4 | fw | CCT GAC ATT CCC AAA GAC AAA GC (SEQ ID NO:33) | 6971.6 | 48 | 64.2 |
| | rv | TCT CTC AAC ACC GCA TAC ACA C (SEQ ID NO:34) | 6569.4 | 50 | 61.2 |
| STAT5A | fw | AAG CCA ACA ATT GCA GCT CTC C (SEQ ID NO:35) | 6649.4 | 50 | 65.0 |
| | rv | GGC AAA CTG AGC TTG GAT CCT C (SEQ ID NO:36) | 6736.4 | 55 | 64.5 |
| STAT5B | fw | CAA GCA TGG GAC TCA GTA GAT CTT G (SEQ ID NO:37) | 7707.0 | 48 | 63.4 |
| | rv | CAG CTG GAG AGC TAC CAT TGT TG (SEQ ID NO:38) | 7080.6 | 52 | 63.4 |
| STAT6 | fw | ATC AGC ACC CTT GAG AGC ATA TAT CAG (SEQ ID NO:39) | 8253.4 | 44 | 64.8 |
| | rv | CTG TGC AGA GAC ACT TGG CCA (SEQ ID NO:40) | 6432.2 | 57 | 64.9 |
| JAK1 | fw | AAT GGC GAG ATC CCC TTG A (SEQ ID NO:41) | 5813.8 | 53 | 62.9 |
| | rv | GCA CCG GCT TTC ATA GAA TCT CT (SEQ ID NO:42) | 6975.6 | 48 | 63.0 |

(continued)

| GENE | Type | Sequence (5' to 3') | MW (μg/ μmol) | %GC | MT (°C) |
|---|---|---|---|---|---|
| JAK2 | fw | TGA TTT TGT GCA CGG ATG GA (SEQ ID NO:43) | 6204.0 | 45 | 63.4 |
| | rv | ACT GCC ATC CCA AGA CAT TCT T (SEQ ID NO:44) | 6615.4 | 45 | 62.1 |
| JAK3 | fw | GCC TGG AGT GGC ATG AGA A (SEQ ID NO:45) | 5918.8 | 58 | 62.4 |
| | rv | CCC CGG TAA ATC TTG GTG AA (SEQ ID NO:46) | 6118.0 | 50 | 62.0 |
| TYK2 | fw | GGG ACC GTG GGC AGG AGC TA (SEQ ID NO:47) | 6249.0 | 70 | 69.0 |
| | rv | GTG CGT GTG GGA GAC CTG GC (SEQ ID NO:48) | 6231.0 | 70 | 68.7 |

The objective of these qPCR reactions is the amplification and quantification of the mRNA molecules that are transcribed from each gene. For that reason, the primers' sequences correspond to exon-exon junctions to assure that no genomic DNA is amplified. Other criterion used in the primers' design were: %GC between 40% and 60% to assure that there is no formation of primer dimers; primer melting temperature between 55 °C and 62 °C because higher temperatures may result in less binding efficiency and lower temperatures may result in less specificity; fw and reverse primers of each gene with similar primer melting temperatures; primer length between 18 and 22 bp for good specificity and binding abilities; and PCR products no longer than 300 bp, since the DNA polymerase cannot amplify DNA fragments longer than this. The exons' sequence of each gene was consulted on, and the primer melting temperature of each primer was consulted on.

The first step of this phase of the procedure was the preparation of a master mix for each RT-PCR reaction containing all the components needed for extension except the template cDNA: 5 μl of SsoAdvanced universal SYBR® Green supermix (2x), and fw and reverse primers at a final concentration of 250-500 nM each. After mixing all these components, 100 ng - 100 fg of template **cDNA** were added, and finally nuclease-free water was also added until 10 μl of reaction volume was reached. The qPCR was then carried out using an Mx3000P Real-Time PCR Detection System (Stratagene) following the PCR program summarized in Table 5. Three experiments were performed for each assay and all samples were run in triplicate.

**Table 5 -** PCR Temperature Cycle Information

| Cycles | Description | Duration of the cycle | Temperature (°C) |
|---|---|---|---|
| 1 | Polymerase activation and cDNA denaturation | 2 min | 95 |
| 35-45 | cDNA denaturation | 5 s | 95 |
| | Annealing, extension and plate read | 30 s | 55-65 |
| 10 | Melt-curve analysis | 10 min | 65-95 gradient |

Relative quantification was performed by determining the RT-PCR signal of the experimental RNA samples in relation to the signal of GAPDH, a housekeeping gene that is used as the internal normalization control gene. The $2(\Delta\Delta C_T)$ method was used to calculate the relative changes in gene expression between HGPS fibroblasts and control fibroblasts, and also between HGPS 9-day 1 μM baricitinib-treated fibroblasts and HGPS mock-treated fibroblasts. In

this method a certain expression level threshold value is established and what is quantified is the number of RT-PCR cycles that it takes for gene expression level to reach the threshold value ($C_T$). For the case of the relative changes in gene expression between HGPS fibroblasts and control fibroblasts, using the 2($\Delta\Delta C_T$) method, equations 1, 2, 3 and 4 are applied for the calculation of the normalized target gene expression. For the relative changes in gene expression between HGPS 9-day 1 μM baricitinib-treated fibroblasts and HGPS mock-treated fibroblasts, the equations are analogous.

$$\Delta C_{T_1} = C_T(Target\ gene\ in\ HGPS\ fibroblasts) - C_T(GAPDH\ in\ HGPS\ fibroblasts) \quad (1)$$

$$\Delta C_{T_2} = C_T(Target\ gene\ in\ control\ fibroblasts) - C_T(GAPDH\ in\ control\ fibroblasts) \quad (2)$$

$$\Delta\Delta C_T = \Delta C_{T_1} - \Delta C_{T_2} \quad (3)$$

$$Normalized\ target\ gene\ expression\ level = 2^{\Delta\Delta C_T} \quad (4)$$

**Example 5 - Baricitinib cell toxicity determination**

Baricitinib short-term (24 h) and long-term (48 h) cytotoxicity at the concentrations of 0.125 μM, 0.25 μM, 0.5 μM, 1 μM, 2 μM, and 4 μM, and DMSO cytotoxicity in cells that are only exposed to the same quantity of DMSO that is used to solubilize baricitinib in all the other concentrations (0.1% v/v) were tested in control fibroblasts (see **Figure 3**) using a CellTox™ Green Cytotoxicity Assay (Promega). To normalize the results, the assay was also performed in control fibroblasts that were grown for 24 h and 48 h without any exposure to baricitinib or DMSO (mock). This assay measures changes in membrane integrity that occur as a result of cell death. It uses a cyanine dye that is excluded from viable cells but preferentially stains the dead cells' DNA. When the dye binds DNA, its fluorescent properties are enhanced.

First, control fibroblasts were harvested in the above mentioned conditions, and after trypsinization the number of cells was counted with a CASY® Cell Counter (Roche). Then, the fibroblasts from each condition were seeded in triplicate in a 96 well plate with 32.000 cells/well in 100 μl of culture media with the appropriate drug concentration, and the plate was incubated at 37 °C for 8 h. After centrifuging the 96 wells plate at 1200 rpm for 5 min, the culture media was aspirated from each well and 100 μl of CellTox™Green Dye Substrate (1:500 in assay buffer) was added. Next, the plate was incubated at room temperature in the dark for 15 min, and the fluorescence was measured in a FLUOstar Omega Microplate Reader (Omega BMG Labtech) using an excitation wavelength of 485 nm and an emission wavelength of 520 nm. This measurement corresponds to the number of dead cells that are present in each well. Following the fluorescence measurement, 4 μl of lysis solution was added to each well, the plate was incubated at 37 °C for 30 min, and the fluorescence level was measured again using the same wavelengths. This last measurement corresponds to the total number of cells present in each well. By dividing the first measurement by the last one, it is possible to obtain the percentage of cell death. Lastly the results were adjusted by considering that the mock treatment corresponds to 0% of cell death.

A further baricitinib cytotoxicity assay was done comparing wild-type mouse fibroblasts and mouse fibroblasts from a progerin mouse model subjected to differing concentrations of The progerin mouse is homozygous for LMNA G609G/G609G (referred to as Δ/ΔG609G/G609G mouse). To determine the non-toxic concentration of baricitinib to be used on mouse fibroblast cultures, cells were treated with different concentration of baricitinib. The results indicate that above 3 μM of baricitinib an increased number of cells in control and progerin-mouse fibroblast were dead. These data indicate that 3 μM of baricitinib should be used for treatment of mouse fibroblast cultures.

**Example 6 - Cumulative population doubling determination**

A cumulative population doubling assay was performed in 4 day and 9 day mock-treated and baricitinib-treated (at the concentrations of 0.25 μM, 0.5 μM, and 1 μM) control and HGPS fibroblasts (see **Figure 4**). First, control and HGPS fibroblasts were cultured at 37 °C and 5% $CO_2$ in culture media without drug. After one week, following trypsinization, the number of cells was counted with a CASY® Cell Counter (Roche). Then, each type of fibroblasts was seeded in triplicate with 150.000 cells/dish at the four different conditions. After 4 days and 9 days, the number of cells harvested in each condition was counted, also using a CASY® Cell Counter (Roche). The cumulative population doubling was determined using equation 5.

$$Cumulative\ Population\ Doubling = 3{,}32 \times \log\left(\frac{cells\ harvested}{cells\ seeded}\right) \qquad (5)$$

A further assay was carried out comparing proliferation of wild-type mouse fibroblasts and mouse fibroblasts from a progerin mouse model treated with 3 μM of baricitinib (see **Figure 15**). These results indicate that 3 μM of baricitinib ameliorate cell growth of control and progerin mouse cells. Cells from progerin (or Δ/Δ) mouse exhibit growth retardation compared to control cells. Baricitinib increases and ameliorate the proliferation potency of the mouse progerin fibroblasts.

**Example 7 - Autophagy measurements in fibroblasts**

Autophagic vacuoles in 4-day and 9-day mock-treated and baricitinib-treated (at the concentrations of 0.25 μM, 0.5 μM, and 1 μM) control and HGPS fibroblasts were quantified using an Autophagy/Cytotoxicity Dual Staining Kit (Cayman Chemical). This assay uses monodansylcadaverine (MDC), a fluorescent compound that is incorporated into multilamellar bodies by both an ion trapping mechanism and the interaction with membrane lipids, as a probe for the detection of autophagic vacuoles in cells.

First, control and HGPS fibroblasts were harvested in the above mentioned conditions and, following trypsinization, the number of cells was counted with a CASY® Cell Counter (Roche). Afterwards, fibroblasts from each condition were seeded in triplicate in a 96 well plate with 32.000 cells/well in 100 μl of culture media with the appropriate drug concentration, and the plate was incubated at 37 °C for 8 h. After centrifuging the 96 well plate at 1200 rpm for 5 min, the culture media was aspirated from each well and 100 μl of staining solution was added. The staining solution was prepared by diluting Cell-Based Monodansylcadaverine 1:1000 in Cell Based Assay Buffer. The assay buffer was prepared by dissolving a Cell-Based Assay Buffer Tablet in 100 ml of distilled water. The plate was then incubated at 37 °C for 10 min and centrifuged for 5 min at 1200 rpm. Next, the supernatant was removed, 100 μl of Cell-Based Assay Buffer was added to each well and the plate was again centrifuged in the same conditions. Lastly, after removing the supernatant, 100 μl of Cell-Based Assay Buffer was added to each well and the autophagic vacuole staining intensity was immediately detected in a FLUOstar Omega Microplate Reader (Omega BMG Labtech) using an excitation wavelength of 355 nm and an emission wavelength of 520 nm. Considering that the autophagy level in mock-treated control fibroblasts is 100%, the level of autophagy relative to mock-treated control fibroblasts for each condition was determined (see **Figure 5**).

Cell-Based Tamoxifen (100 mM), a known inducer of autophagy, was used as a positive control. This compound was initially added to a control culture diluted 1:50.000 into the culture media. Cell Based Assay Buffer was used as a negative blank control.

A further assay was carried out showing autophagy levels in wild-type mouse fibroblasts and mouse fibroblasts from a progerin mouse model (see **Figure 16**). The results indicate that 3 μM baricitinib increases the levels of autophagy in both control and progerin fibroblasts. Importantly, the levels are increased by an average of 39.9% in progerin mouse fibroblast relative to the mock-treated control cells

**Example 8 - 20S Proteasome activity measurement in fibroblasts**

20S proteasome core particle in 4-day and 9-day mock-treated and baricitinib-treated (at the concentrations of 0.25 μM, 0.5 μM, and 1 μM) control and HGPS fibroblasts was quantified using a 20S Proteasome Assay Kit (Cayman Chemical). This assay uses a specific 20S substrate (SUC-LLVY-AMC) which, upon cleavage by the active enzyme, generates a fluorescent product that can be detected.

First, control and HGPS fibroblasts were harvested in the above mentioned conditions and, following trypsinization, the number of cells was counted with a CASY® Cell Counter (Roche). The fibroblasts from each condition were seeded in triplicate in a 96 well plate with 100.000 cells/well in 100 μl of culture media with the appropriate drug concentration, and the plate was incubated at 37 °C for 8 h. After centrifuging the 96 well plate at 1200 rpm for 5 min, the culture media was aspirated and 200 μl of 20S Proteasome Assay Buffer was added to each well. Next, the plate was again centrifuged in the same conditions, the supernatant was aspirated, and 100 μl of 20S Proteasome Lysis Buffer was added to each well. Then, the plate was incubated with gentle shaking for 30 min at room temperature to allow cell lysis and it was centrifuged at 1300 rpm for 7 min. Following centrifugation, 90 μl of the supernatant from each well was

transferred to a new empty well together with 10 μl of substrate solution. The substrate solution was prepared by adding 25 μl of 20S Proteasome Substrate (SUC-LLVY-AMC) to 1 ml of 20S Proteasome Assay Buffer. Lastly, the plate was incubated at 37 °C for 1 h and after that time the fluorescence intensity was read in FLUOstar Omega Microplate Reader (Omega BMG Labtech) using an excitation wavelength of 360 nm and an emission wavelength of 480 nm. Considering that the proteasomal activity in mock-treated control fibroblasts is 100%, the proteasomal activity relative to mock-treated control fibroblasts was calculated for each condition.

A Jurkat cell lysate supernatant which contains a high level of 20S activity was used as positive control (20S Proteasome Positive Control) and 20S Proteasome Assay Buffer was used as negative blank control.

### Example 9 - Intracellular ROS measurement in fibroblasts

Intracellular ROS in 4-day and 9-day mock-treated and baricitinib-treated (at the concentrations of 0.25 μM, 0.5 μM, and 1 μM) control and HGPS fibroblasts was measured using a DCFDA Cellular ROS Detection Assay Kit (Abcam). This assay uses 2',7'-dichlorofluorescein diacetate (DCFDA), a cell permeant reagent that after entering the cell is deacetylated by cellular esterases to a non-fluorescent compound that is later oxidized by ROS into 2',7'-dichlorofluorescein (DCF). DCF is highly fluorescent and can be detected by fluorescence spectroscopy.

First, control and HGPS fibroblasts were harvested in the above mentioned conditions and, following trypsinization, the number of cells was counted with a CASY® Cell Counter (Roche). The fibroblasts from each condition were seeded in triplicate in a 96 well plate with 32.000 cells/well in 100 μl of culture media with the appropriate drug concentration, and the plate was incubated at 37 °C for 8 h. After the incubation, the culture media was aspirated, 100 μl of 1 x Buffer were added to each well, and the plate was centrifuged at 1200 rpm for 5 min. Then, the supernatant was aspirated, 100 μl of DCFDA solution was added to each well and the plate was incubated at 37 °C for 45 min in the dark. The DCFDA solution was prepared by adding 12.5 μl of 20 mM DCFDA solution to 10 ml of 1x Buffer. Next, the plate was centrifuged under the same conditions, the supernatant was aspirated, and 100 μl of 1x Buffer was added to each well. Following the addition of 1x Buffer, fluorescence was measured immediately in a FLUOstar Omega Microplate Reader (Omega BMG Labtech) using an excitation wavelength of 485 nm and an emission wavelength of 535 nm. Considering that the ROS level in mock-treated control fibroblasts is 100%, the ROS levels relative to mock-treated control fibroblasts were calculated for each condition.

Cells treated with 50 μM tert-butyl hydrogen peroxide (TBHP), a compound that mimics ROS activity to oxidize DCFDA to fluorescent DCF, were used as a positive control. Culture media with DCFDA and without cells was used as a blank negative control to assure that the media does not affect DCFDA reading.

### Example 10 - Intracellular ATP measurement in fibroblasts

The intracellular ATP content of 4-day and 9-day mock-treated and baricitinib-treated (at the concentrations of 0.25 μM, 0.5 μM, and 1 μM) control and HGPS fibroblasts was measured by using a CellTiter-Glo® Luminescent Cell Viability Assay (Promega). This assay relies in the properties of a proprietary thermostable luciferase which generates a stable luminescent signal that is proportional to the amount of ATP in the cells by catalyzing the mono-oxygenation of luciferin in the presence of $Mg^{2+}$, ATP and molecular oxygen.

First, control and HGPS fibroblasts were harvested in the above mentioned conditions and the number of cells was counted with a CASY® Cell Counter (Roche). Then, the fibroblasts from each condition were seeded in triplicate in a 96 well plate with 32.000 cells/well in 100 μl of culture media with the appropriate drug concentration, and the plate was incubated at 37 °C for 8 h. After equilibrating the 96 well plate at room temperature for 30 min and centrifuging it at 1200 rpm for 5 min, the culture media was aspirated and 100 μl of CellTiter-Glo® Reagent was added to each well. This reagent is obtained by transferring 10 ml of CellTiter-Glo® Buffer into an amber bottle containing CellTiter-Glo® Substrate and then mixing it gently by vortexing. The plate was then mixed for 2 min in an orbital shaker to induce cell lysis and following that, it was incubated at room temperature for 10 min to stabilize the luminescent signal. Finally, luminescence was recorded in a FLUOstar Omega Microplate Reader (Omega BMG Labtech). Considering that the ATP level in mock-treated control fibroblasts is 100%, the ATP levels relative to mock-treated control fibroblasts were calculated for each condition.

Culture media without cells was used as a blank negative control to assure there is no interference with the luciferase reaction.

### Example 11 - Immunocytochemistry

An immunocytochemistry assay with an anti-progerin antibody was performed in control and HGPS fibroblasts to substantiate that HGPS cells are characterized by an accumulation of progerin in the nucleus. Both control and HGPS fibroblasts were directly grown in coverslips. After one day of growing, the fibroblasts were washed with PBS (Sigma)

for three times and fixed with 4% PFA (Sigma-Aldrich) at room temperature for 15 min to preserve cellular morphology and cellular structures in their native conformation. Then, the fibroblasts were washed again for three times with PBS (Sigma) and permeabilized by 1% Triton X-100 (PanReac AppliChem) for 3 min at room temperature. By permeabilizing the fibroblasts, intracellular structures become accessible for antibodies. Following the permeabilization, fibroblasts were again washed for three times with PBS (Sigma) and blocked with 15% FBS/PBST blocking buffer at room temperature for 30 min to minimize unspecific binding of the primary antibody inside the cell. The blocking buffer contains 15% FBS (Gibco by life technologies) and 85% PBST, while PBST is constituted by 98.5% PBS (Sigma) and 1.5% Tween 20 (Sigma). Next, the fibroblasts were incubated in anti-progerin S9 primary antibody (McClintock *et al.*, 2007), 1 μg/ml diluted in blocking buffer, for 24h at room temperature. Subsequent to the primary antibody incubation, fibroblasts were washed three times with PBST for 5 min to remove the primary antibody that did not bind to any protein inside the cell and incubated in Alexa Fluor® 555 donkey anti-rabbit IgG (H+L) secondary antibody (Thermo Fisher Scientific), 1/800 diluted in blocking buffer, for 1h at room temperature. After this, fibroblasts were washed for three times with PBST for 5 min to remove the secondary antibody that did not bind to the primary antibody inside the cell and, afterwards, washed for three times with PBS (Sigma) for 5 min. Finally, the samples were counterstained with the DNA dye DAPI in Vectashield mounting medium (Vector Inc.) and observed using an Axioplan fluorescence microscope (Carl Zeiss).

**Example 12 - Western blot analysis**

A quantitative Western Blot analysis using a Santa Cruz rabbit anti-lamin A/C (H-1 10) antibody was performed in control and HGSP fibroblasts that were mock-treated or baricitinib-treated (at the concentrations of 0.25 μM, 0.5 μM or 1 μM) for a period of 4 and 9 days to determine if baricitinib can reduce progerin levels in HGPS fibroblasts (see **Figures 9, 10,** and **13**). A similar quantitative Western Blot analysis was carried out in wild-type mouse fibroblasts compared to fibroblasts obtained from the progerin mouse model (see **Figure 17**).

**Preparation of cell pellets and gel electrophoresis loading samples**

First, control and HGPS fibroblasts were harvested in the above mentioned conditions, and after trypsinization the number of cells was counted with a CASY® Cell Counter (Roche). Subsequently, the cell suspension was centrifuged for 5 min at 1200 rpm, the supernatant was aspirated, and the cells were washed with 4.5 ml PBS (Sigma). These steps were performed thrice in order to assure that all culture media is removed. Then, the cells were transferred to Eppendorf tubes with 1 ml PBS (Sigma) and the tubes were centrifuged for 5 min at 1200 rpm. After fully aspirating PBS, the cell pellets were obtained. Following the generation of cell pellets, the next action was the preparation of the gel electrophoresis loading samples. The first step was the addition of 100 μl per $10^7$ cells of Laemmli Buffer-inhibitor mix to each Eppendorf tube to lyse the fibroblasts and denature the proteins of the lysate. This solution contains 60 μl of β-mercaptoethanol (Biorad) to reduce the intra and inter-molecular disulfide bonds, 12.5 μl of protease inhibitor cocktail (Biorad) and 7.5 μl of 200 mM PMSF (Biorad) to avoid protein degradation, and 0.95 ml of Laemmli Sample buffer (BioRad), a gel electrophoresis loading buffer that contains the SDS detergent that denatures the proteins and gives them an overall negative charge. Afterwards, the solution in each Eppendorf was vortexed for 2 min and heated at 99 °C for 3 minutes. These two steps were repeated for three times to assure that all the cells were lysed.

**Determination of the total protein concentration of the gel electrophoresis loading samples**

The total protein concentration of each gel electrophoresis loading sample was determined by using a series of dilutions of known concentrations of bovine serum albumin (BSA) to obtain a standard curve. To begin with, the content of an Albumin Standard Ampule that contains BSA at 2 mg/ml (Sigma-Aldrich) was diluted in Laemmli Buffer-inhibitor mix into the concentrations of 2000 μg/ml, 1500 μg/ml, 1000 μg/ml, 750 μg/ml, 500 μg/ml, 250 μg/ml, 125 μg/ml, 25 μg/ml and 0 μg/ml to obtain the BSA standard dilutions samples that were used to acquire the standard curve. Subsequently, 2 μl of each gel electrophoresis loading sample is diluted in 8 μl of Laemmli Sample buffer (BioRad). Then, 2 μl of each BSA standard dilution sample and 2 μl of each diluted electrophoresis loading sample were blotted for three times on the nitrocellulose blotting membrane. After being washed with $ddH_2O$, the membrane was incubated with ponceau S (Sigma-Aldrich) for 5 min in order to stain the proteins that are present in the membrane, and finally washed again with $ddH_2O$ to remove the dye that did not bind to any protein. A picture of the blotting membrane was then taken utilizing a ChemiDoc™ MP Imaging System (Biorad), and using that picture it was possible to establish a standard curve through the medium of the software Image Lab (Biorad). The total protein concentration of each gel electrophoresis loading sample was calculated based on this standard curve. Using the total protein concentrations, a final gel electrophoresis loading sample solution of 20 μl containing 30 μg of total protein was prepared for each sample.

### SDS-gel electrophoresis and protein transfer to a nitrocellulose membrane

A SDS-gel electrophoresis to separate the proteins in each sample based on their molecular weight was performed in a 4-20% Mini-Protean® TGX™ Precast Protein Gel (BioRad), using 1x running buffer, at 200 V until the 37 kD lane reached the bottom of the gel. This running buffer was prepared as 10x running buffer that contains 1.92 M glycine (Carl Roth), 248 mM tris-base (Sigma) and 1% SDS (Sigma), and then diluted in $ddH_2O$ to 1 x. Before being added to the wells of the gel (20 μl per well), each sample was vortexed for three times during 30 s and heated at 99 °C for 3 minutes to completely denature the proteins that are present in each sample. 5 μl of Precision plus protein standard (Biorad) were added to one of the wells to be used as a marker. After finishing the SDS-gel electrophoresis, the gel was removed from the cassette, washed with $ddH_2O$ and equilibrated for 10 min in transfer buffer. The transfer buffer contains 20 mM tris (Sigma), 150 mM glycine (Carl Roth), 20% methanol (PanReac AppliChem) and 0.1% SDS (Sigma). The proteins that were separated in the gel were then transferred to a nitrocellulose membrane using a Trans-Blot® Turbo™ Transfer System (Biorad) at 25 V for 20 min, and using the same transfer buffer. The fiber pads, filter papers and nitrocellulose membrane that were used in the electroblotting were previously equilibrated overnight in pre-cooled transfer buffer at 4 °C. Even before that, the nitrocellulose membrane had to be equilibrated in $ddH_2O$ for 10 min. At the end of the electroblotting procedure, the nitrocellulose membrane was washed with $ddH_2O$ and incubated in Ponceau S (Sigma-Aldrich) for 5 min to verify the effectiveness of the protein transfer from the gel to the membrane. Following the Ponceau S incubation, the membrane was again washed with $ddH_2O$ and a picture of it was taken using a ChemiDoc™ MP Imaging System (Biorad). Ponceau S staining technique is reversible to allow further immunological detection. Accordingly, after this procedure the blotting membrane was washed with PBST and destained in PBS (Sigma). PBST contains 98.5% PBS (Sigma) and 1.5% Tween 20 (Sigma).

### Blocking and immunodetection

Before proceeding to the immunodetection, the membrane had to be blocked to avoid unspecific binding of the antibody used in the immunodetection. For blocking, the nitrocellulose membrane was incubated for 4 h in milk buffer. The milk buffer contains 1.2% nonfat dry milk vitamin A+D fortified (Nestlé) and 98.8% PBST. PBST contains 98.5% PBS (Sigma) and 1.5% Tween 20 (Sigma). Having completed the blocking, the membrane was incubated in rabbit anti-lamin A/C H-1 10 antibody (Santa Cruz Biotechnology, 1/10.000 in PBST) for 12 h at 4 °C. Following that incubation period, to remove the primary antibody that did not bind to any protein, the membrane was washed with PBST for three times during 5 min and subsequently incubated in Peroxidase-conjugated AffiniPure goat anti-rabbit IgG (H+L) (Abcam, 1/5000 in PBST) for 50 min at room temperature. Next, the membrane was again washed with PBST for three times during 5 min and then washed with PBS (Sigma) for three times during 5 min to remove the secondary antibody that did not bind to any primary antibody. Finally, the proteins were visualized using ECL1 and ECL2 (Biorad) at a dilution of 1:1 in a ChemiDoc™ MP Imaging System (Biorad). Protein signals were quantified by normalizing to β-tubulin using the software Image Lab (Biorad).

### Example 13 - Statistical analyses

In all the experiments, the results are presented as the mean ± SD. Comparisons were performed using Student's t-test. A P value of $P < 0.05$ was considered statistically relevant.

### REFERENCES

Alten, R. and Nitschmann, S. (2017). Efficacy and safety of baricitinib in Japanese patients with rheumatoid arthritis: Subgroup analyses of four multinational phase 3 randomized trials. Internist (Berl) 58, 1341-1344.

Carfagna, M., Cannady, E., Ryan, T., Herman, J., Truex, L., Narwani, K. and Sullivan, J. (2017). Carcinogenicity assessment of baricitinib in Tg.rasH2 mice and Sprague-Dawley (Crl:CD) rats. Regulatory in Toxicology and Pharmacology.

Fleischmann, R., Alam, J., Arora, V., Bradley, J., Schlichting, D. E., Muram, D. and Smolen, J. S. (2017). Safety and efficacy of baricitinib in elderly patients with rheumatoid arthritis. RMD Open 3, e000546.

Genovese, M. C. et al. (2016). Baricitinib in Patients with Refractory Rheumatoid Arthritis. New England Journal of Medecin 374, 1243-1252.

Jabbari, A. et al. (2015). Reversal of Alopecia Areata Following Treatment With the JAK1/2 Inhibitor Baricitinib. EBioMedicine 2, 351-355.

Karonitsch, T. et al. (2017). Targeted inhibition of Janus kinases abates interfon gamma-induced invasive behaviour

of fibroblast-like synoviocytes. Rheumatolog.
Keystone, E. C., Genovese, M. C., Schlichting, D. E., de la Torre, I., Beattie, S. D., Rooney, T. P. and Taylor, P. C. (2017a). Safety and Efficacy of Baricitinib Through 128 Weeks in an Open-label, Longterm Extension Study in Patients with Rheumatoid Arthritis. Journal of Rheumatology 15.
Keystone, E. C. et al. (2015). Safety and efficacy of baricitinib at 24 weeks in patients with rheumatoid arthritis who have had an inadequate response to methotrexate. Annual Rhrumatology disease 74, 333-340.
Keystone, E. C. et al. (2017b). Patient-reported outcomes from a phase 3 study of baricitinib versus placebo or adalimumab in rheumatoid arthritis: secondary analyses from the RA-BEAM study. Annual Rhreumatology disease 76, 1853-1861.
Kubo, S., Nakayamada, S. and Tanaka, Y. (2016). Baricitinib for the treatment of rheumatoid arthritis. Expert Review in Clinical Immunology. 12, 911-919.
Lee, Y. H. and Bae, S. C. (2017). Comparative efficacy and safety of baricitinib 2 mg and 4 mg in patients with active rheumatoid arthritis : A Bayesian network meta-analysis of randomized controlled trials. Z Rheumatology.
Markham, A. (2017). Baricitinib: First Global Approval. Drugs 77, 697-704.
Murakami, K. et al. (2017). A Jak1/2 inhibitor, baricitinib, inhibits osteoclastogenesis by suppressing RANKL expression in osteoblasts in vitro. PLoS ONE 12, e0181126.
Nakayamada, S., Kubo, S., Iwata, S. and Tanaka, Y. (2016). Recent Progress in JAK Inhibitors for the Treatment of Rheumatoid Arthritis. BioDrug 30, 407-419.
Norman, P. (2014). Expert Opinion Investigative Drugs 23, 1067-1077.
Osorio F. G. et al (2011). Splicing-Directed Therapy in a New Mouse Model of Human Accelerated Aging. Sci Transl Med. Oct 26;3(106):106ra107
Shi, J. G., Chen, X., Lee, F., Emm, T., Scherle, P. A., Lo, Y., Punwani, N., Williams, W. V. and Yeleswaram, S. (2014). The pharmacokinetics, pharmacodynamics, and safety of baricitinib, an oral JAK 1/2 inhibitor, in healthy volunteers. Journal of Clinical Pharmacology 54, 1354-1361.
Tanaka, Y., Emoto, K., Cai, Z., Aoki, T., Schlichting, D., Rooney, T. and Macias, W. (2016). Efficacy and Safety of Baricitinib in Japanese Patients with Active Rheumatoid Arthritis Receiving Background Methotrexate Therapy: A 12-week, Double-blind, Randomized Placebo-controlled Study. Journal of Rheumatology 43, 504-514.
Taylor, P. C. et al. (2017). Baricitinib versus Placebo or Adalimumab in Rheumatoid Arthritis. New England Journal of Medecin 376, 652-662.
Yamaoka, K. (2016). Janus kinase inhibitors for rheumatoid arthritis. Current Opinion in Chemistry and Biology 32, 29-33.

## Claims

1. Compound for use in treating Hutchinson-Gilford progeria syndrome in a subject, wherein the compound selectively and reversely inhibits the JAK-STAT pathway in the subject.

2. Pharmaceutical composition comprising a compound capable of selectively and reversely inhibiting the JAK-STAT pathway in a subject and a pharmaceutically acceptable carrier for use in treating Hutchinson-Gilford progeria syndrome in a subject.

3. The compound for use of claim 1 or pharmaceutical composition for use of claim 2, wherein the compound selectively and reversely inhibits JAK1 and/or JAK2.

4. The compound for use of claim 1 or pharmaceutical composition for use of claim 2, wherein the compound exhibits an $IC_{50}$ for JAK1 of about 6 nM or less, and wherein the compound exhibits an $IC_{50}$ for JAK2 of about 6 nM or less.

5. The compound for use of claim 1 or pharmaceutical composition for use of claim 2, wherein the compound is baricitinib having the following structure:

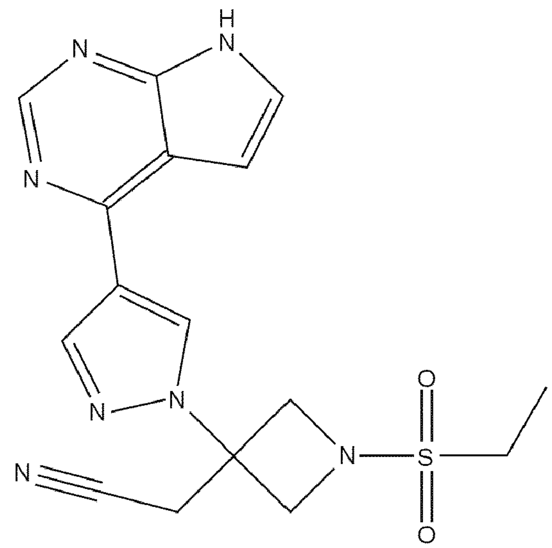

,

or a pharmaceutically acceptable salt thereof.

6. The compound for use of claim 1 or pharmaceutical composition for use of claim 2, wherein treatment is **characterized by** amelioration of alopecia, arthritis, vascular disease, and lipodystrophy.

FIG. 1

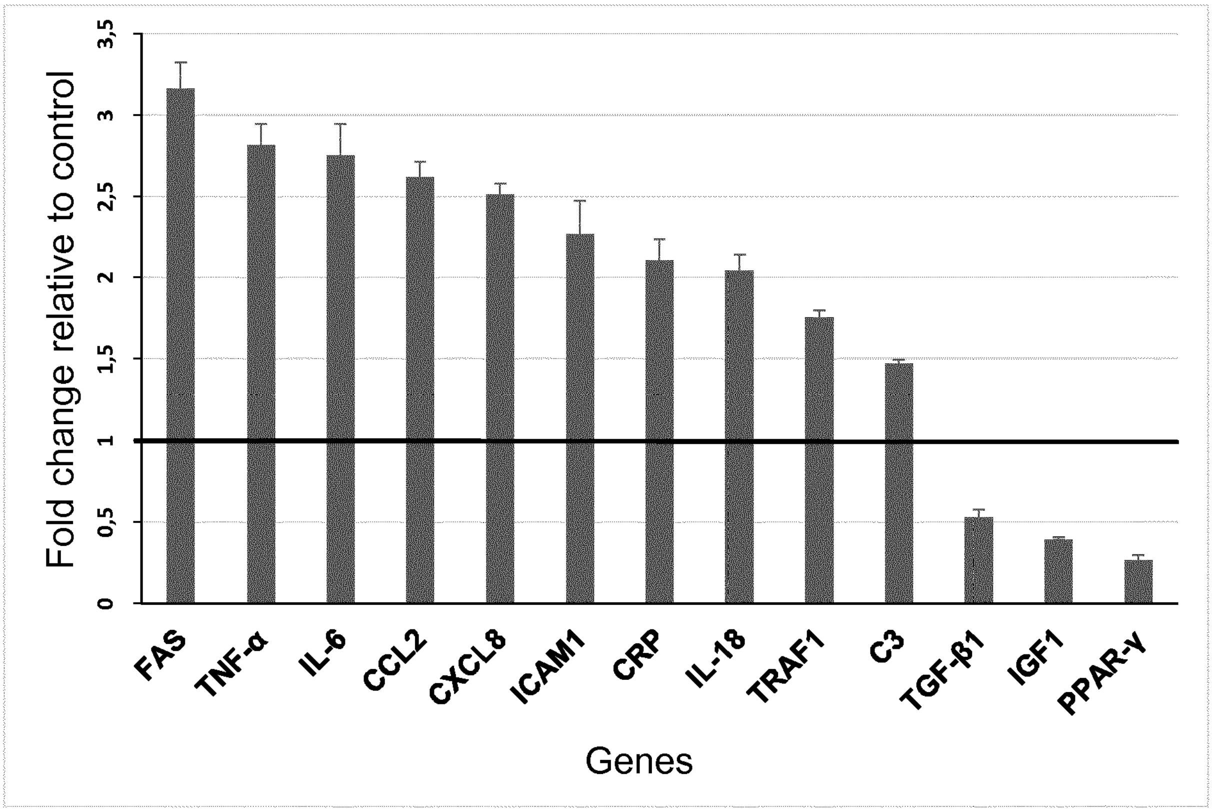
Fold change relative to control
0
0,5
1
1,5
2
2,5
3
3,5
FAS
TNF-α
IL-6
CCL2
CXCL8
ICAM1
CRP
IL-18
TRAF1
C3
TGF-β1
IGF1
PPAR-γ
Genes

FIG. 2

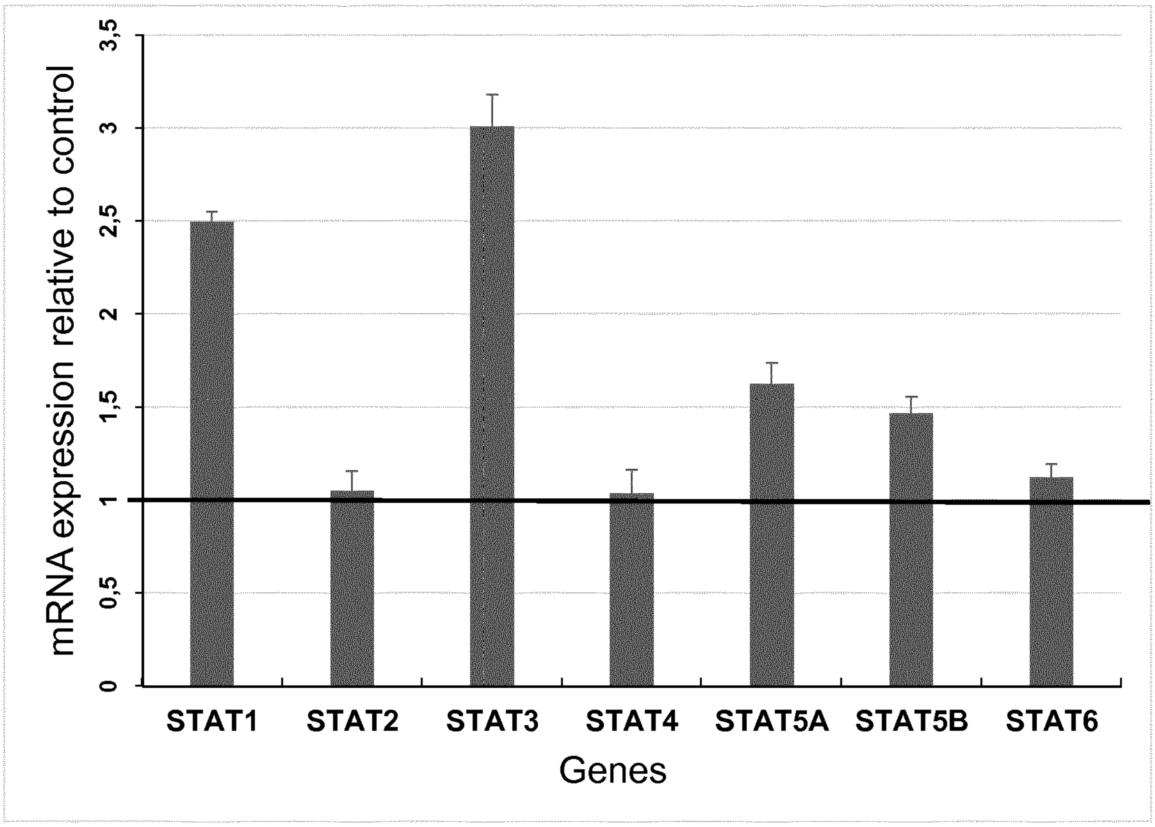
mRNA expression relative to control
0
0,5
1
1,5
2
2,5
3
3,5
STAT1
STAT2
STAT3
STAT4
STAT5A
STAT5B
STAT6
Genes

FIG. 3

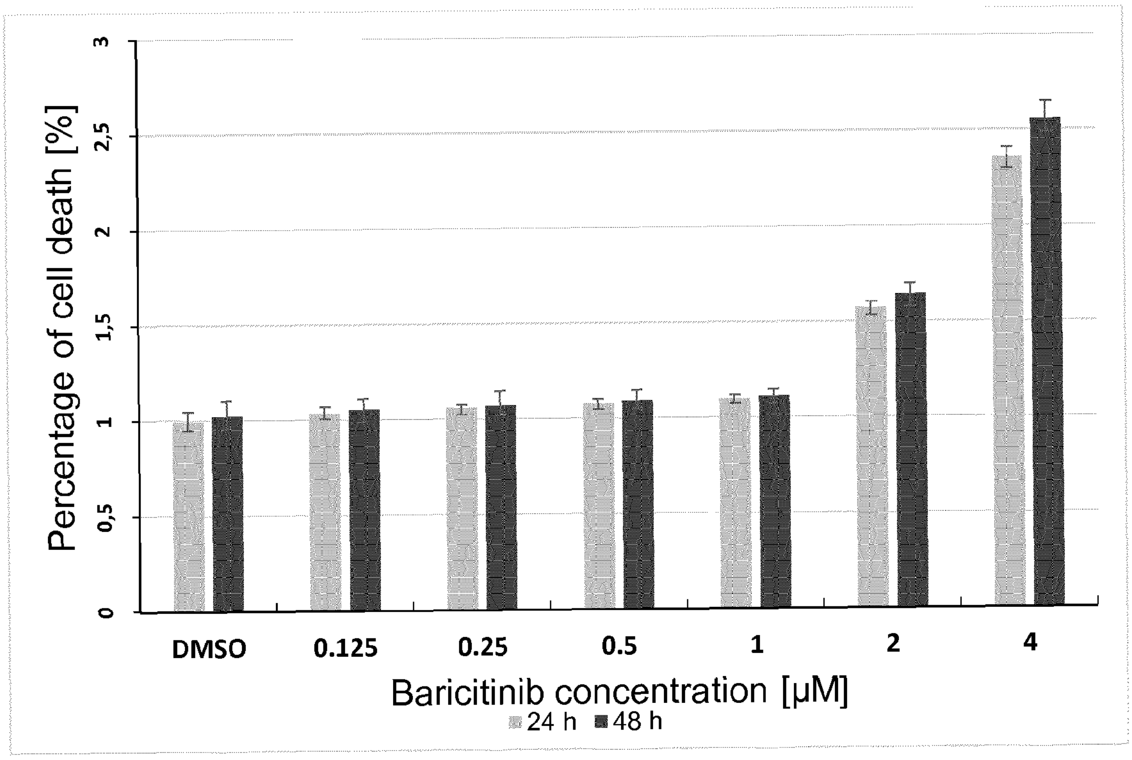
Percentage of cell death [%]
0
0,5
1
1,5
2
2,5
3
DMSO
0.125
0.25
0.5
1
2
4
Baricitinib concentration [µM]
24 h
48 h

FIG. 4

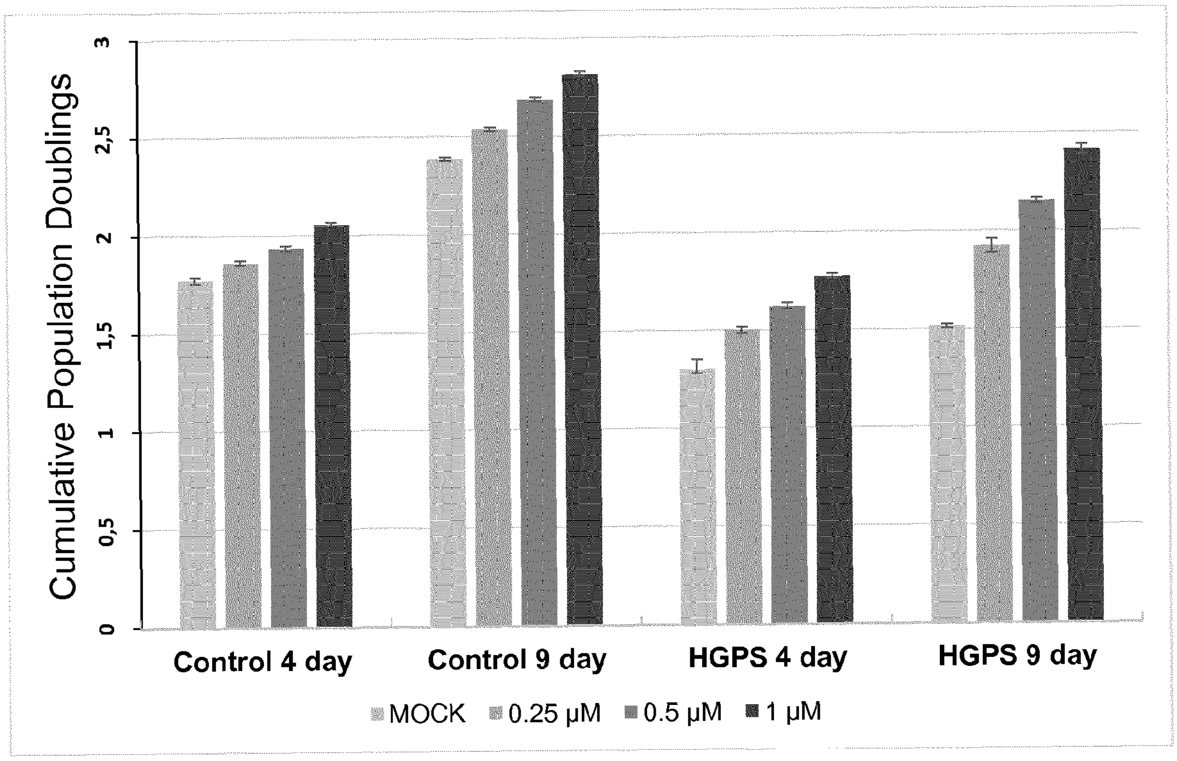
Cumulative Population Doublings
0
0,5
1
1,5
2
2,5
3
Control 4 day
Control 9 day
HGPS 4 day
HGPS 9 day
MOCK
0.25 µM
0.5 µM
1 µM

FIG. 5

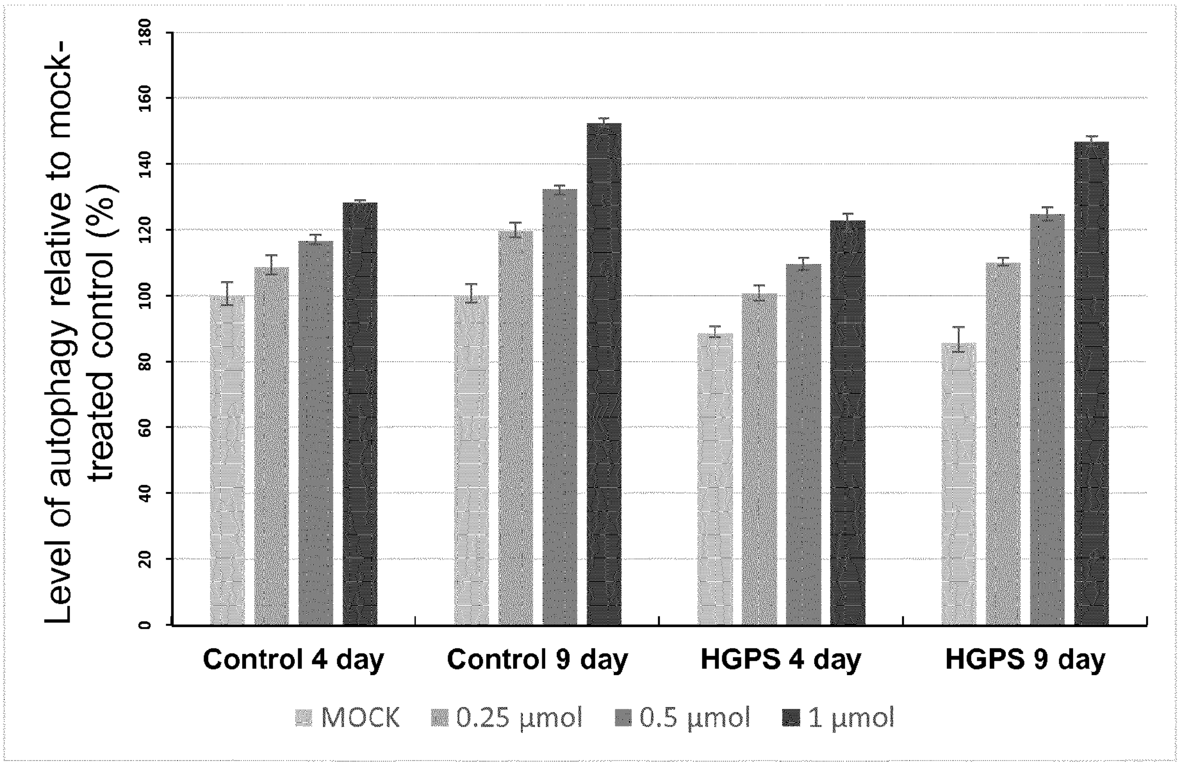
Level of autophagy relative to mock-treated control (%)
0
20
40
60
80
100
120
140
160
180
Control 4 day
Control 9 day
HGPS 4 day
HGPS 9 day
MOCK
0.25 µmol
0.5 µmol
1 µmol

FIG. 6

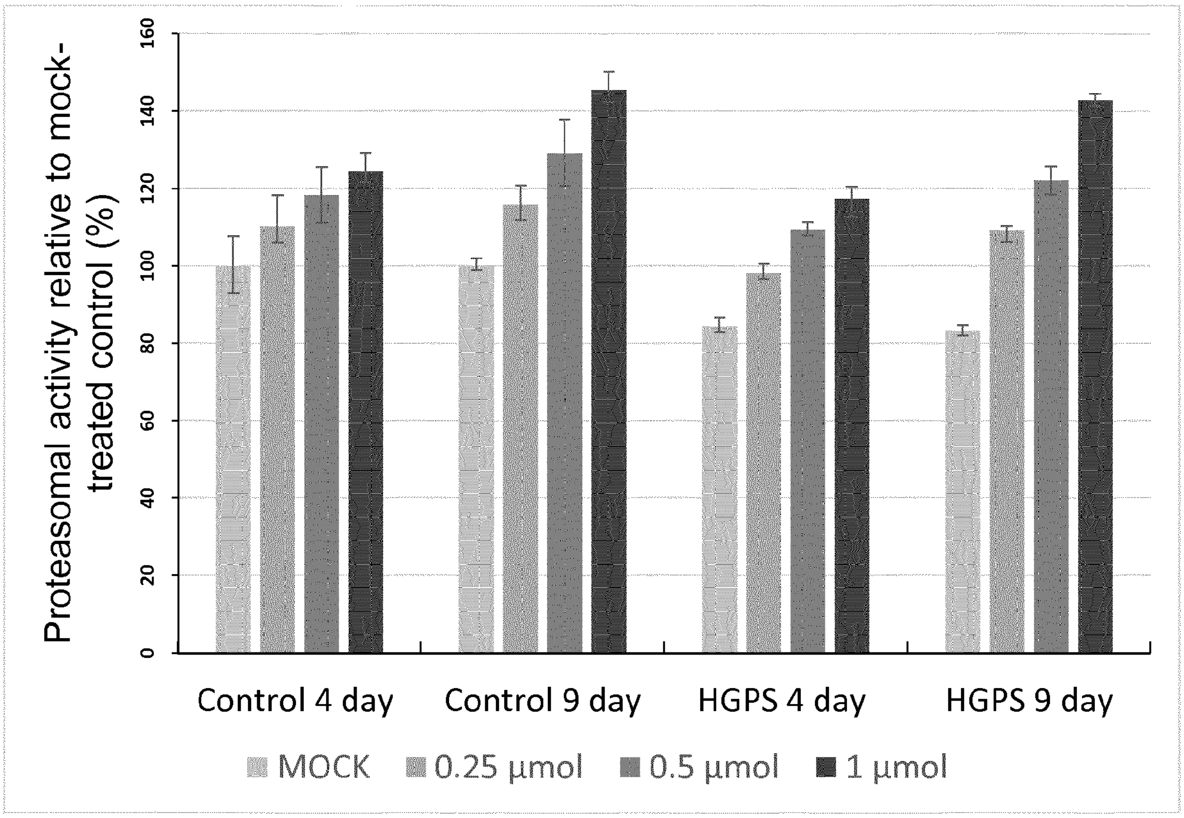
Proteasomal activity relative to mock-treated control (%)
0
20
40
60
80
100
120
140
160
Control 4 day
Control 9 day
HGPS 4 day
HGPS 9 day
MOCK
0.25 µmol
0.5 µmol
1 µmol

FIG. 7

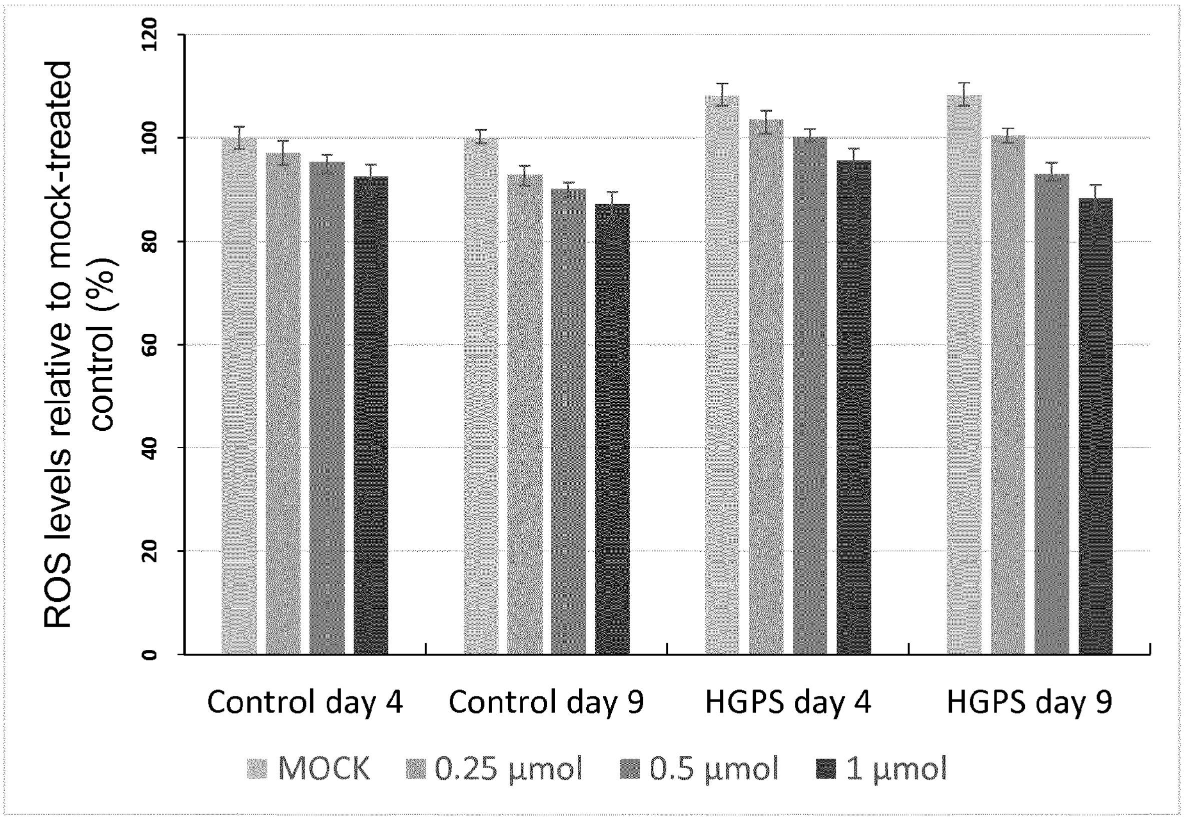
ROS levels relative to mock-treated control (%)
0
20
40
60
80
100
120
Control day 4
Control day 9
HGPS day 4
HGPS day 9
MOCK
0.25 µmol
0.5 µmol
1 µmol

FIG. 8

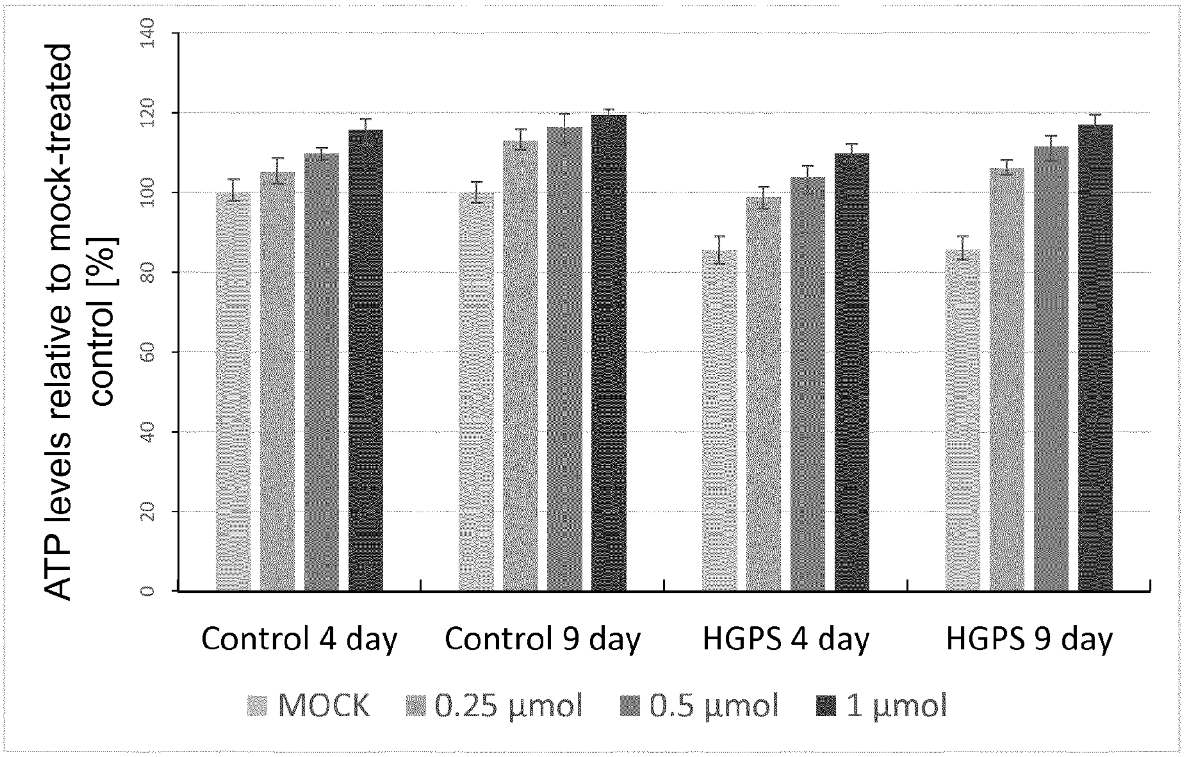
ATP levels relative to mock-treated control [%]
0
20
40
60
80
100
120
140
Control 4 day
Control 9 day
HGPS 4 day
HGPS 9 day
MOCK
0.25 µmol
0.5 µmol
1 µmol

FIG. 9

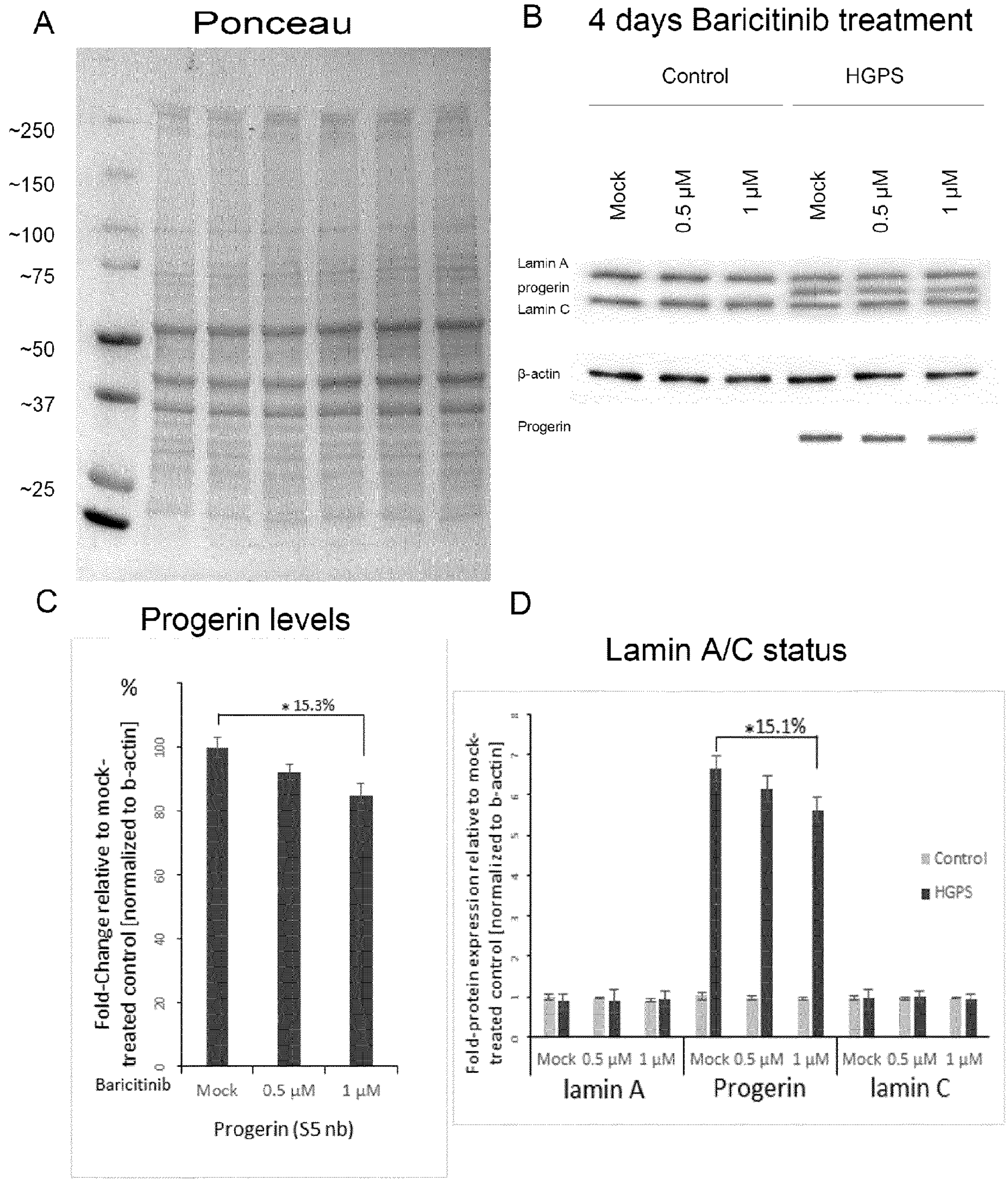
A
Ponceau
~250
~150
~100
~75
~50
~37
~25
B
4 days Baricitinib treatment
Control
HGPS
Mock
0.5 μM
1 μM
Mock
0.5 μM
1 μM
Lamin A
progerin
Lamin C
β-actin
Progerin
C
Progerin levels
%
Fold-Change relative to mock-treated control [normalized to b-actin]
* 15.3%
Baricitinib
Mock
0.5 μM
1 μM
Progerin (S5 nb)
D
Lamin A/C status
Fold-protein expression relative to mock-treated control [normalized to b-actin]
*15.1%
Control
HGPS
Mock 0.5 μM 1 μM
Mock 0.5 μM 1 μM
Mock 0.5 μM 1 μM
lamin A
Progerin
lamin C

FIG. 10

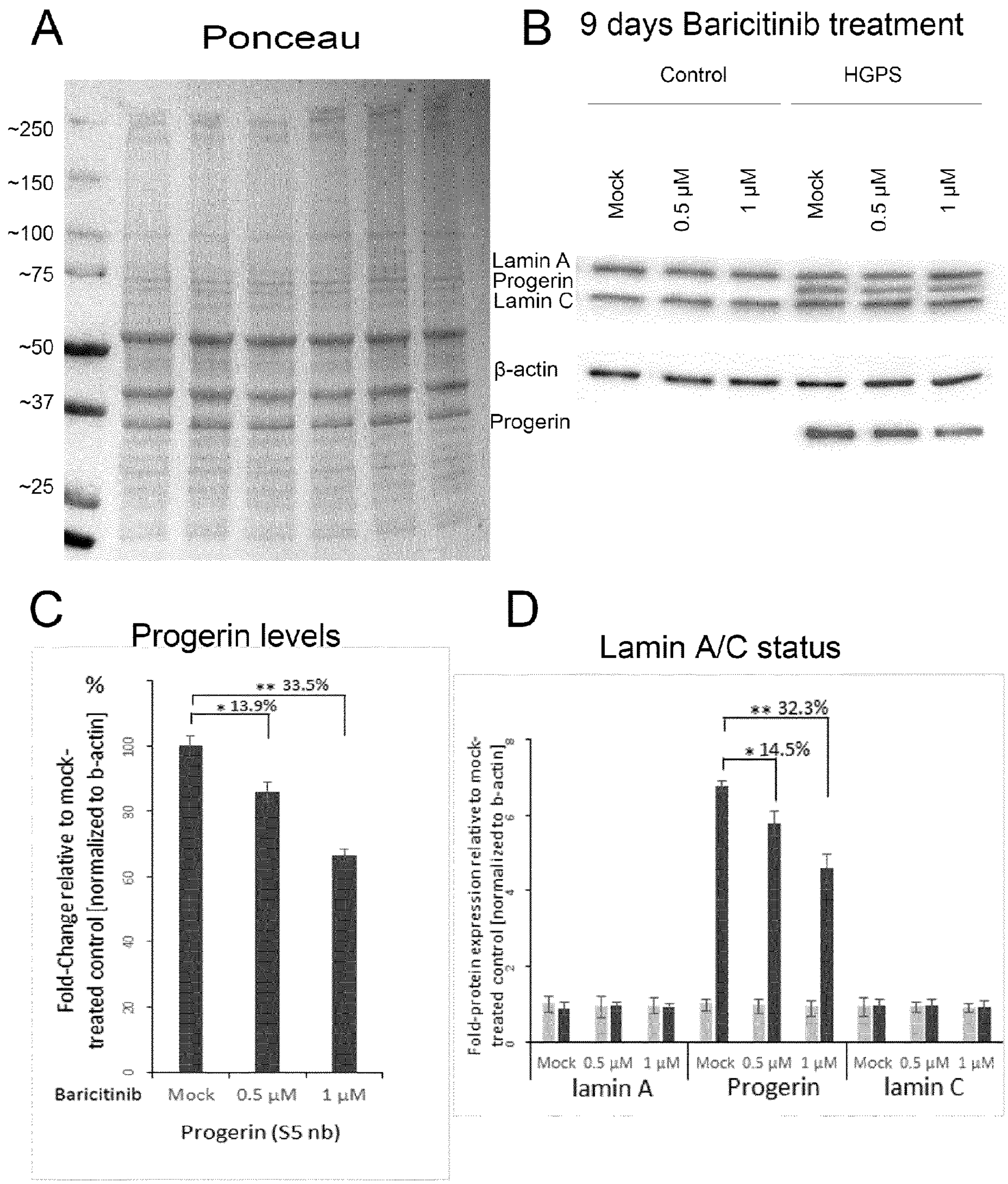
A
Ponceau
~250
~150
~100
~75
~50
~37
~25
B
9 days Baricitinib treatment
Control
HGPS
Mock
0.5 µM
1 µM
Mock
0.5 µM
1 µM
Lamin A
Progerin
Lamin C
β-actin
Progerin
C
Progerin levels
%
** 33.5%
* 13.9%
Fold-Change relative to mock-treated control [normalized to b-actin]
Baricitinib
Mock
0.5 µM
1 µM
Progerin (S5 nb)
D
Lamin A/C status
** 32.3%
* 14.5%
Fold-protein expression relative to mock-treated control [normalized to b-actin]
Mock 0.5 µM 1 µM
lamin A
Mock 0.5 µM 1 µM
Progerin
Mock 0.5 µM 1 µM
lamin C

FIG. 11

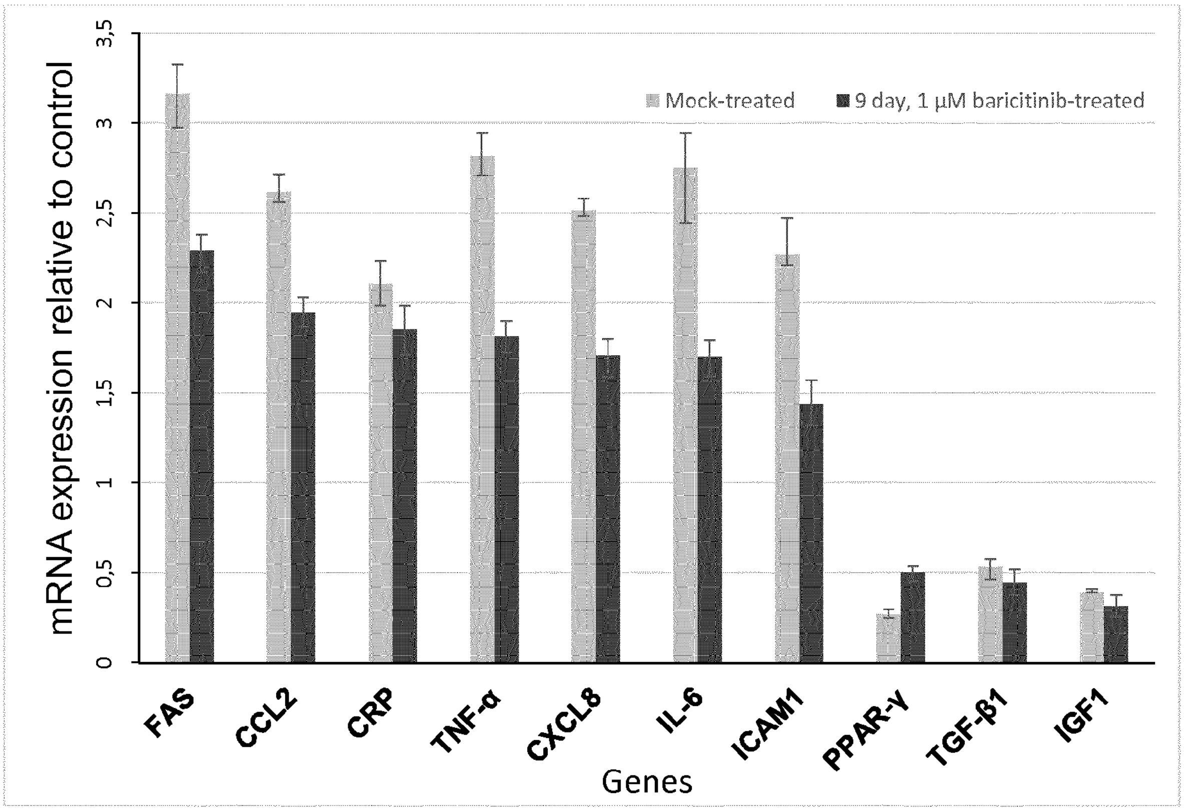
mRNA expression relative to control
0
0,5
1
1,5
2
2,5
3
3,5
Mock-treated
9 day, 1 µM baricitinib-treated
FAS
CCL2
CRP
TNF-α
CXCL8
IL-6
ICAM1
PPAR-γ
TGF-β1
IGF1
Genes

FIG. 12

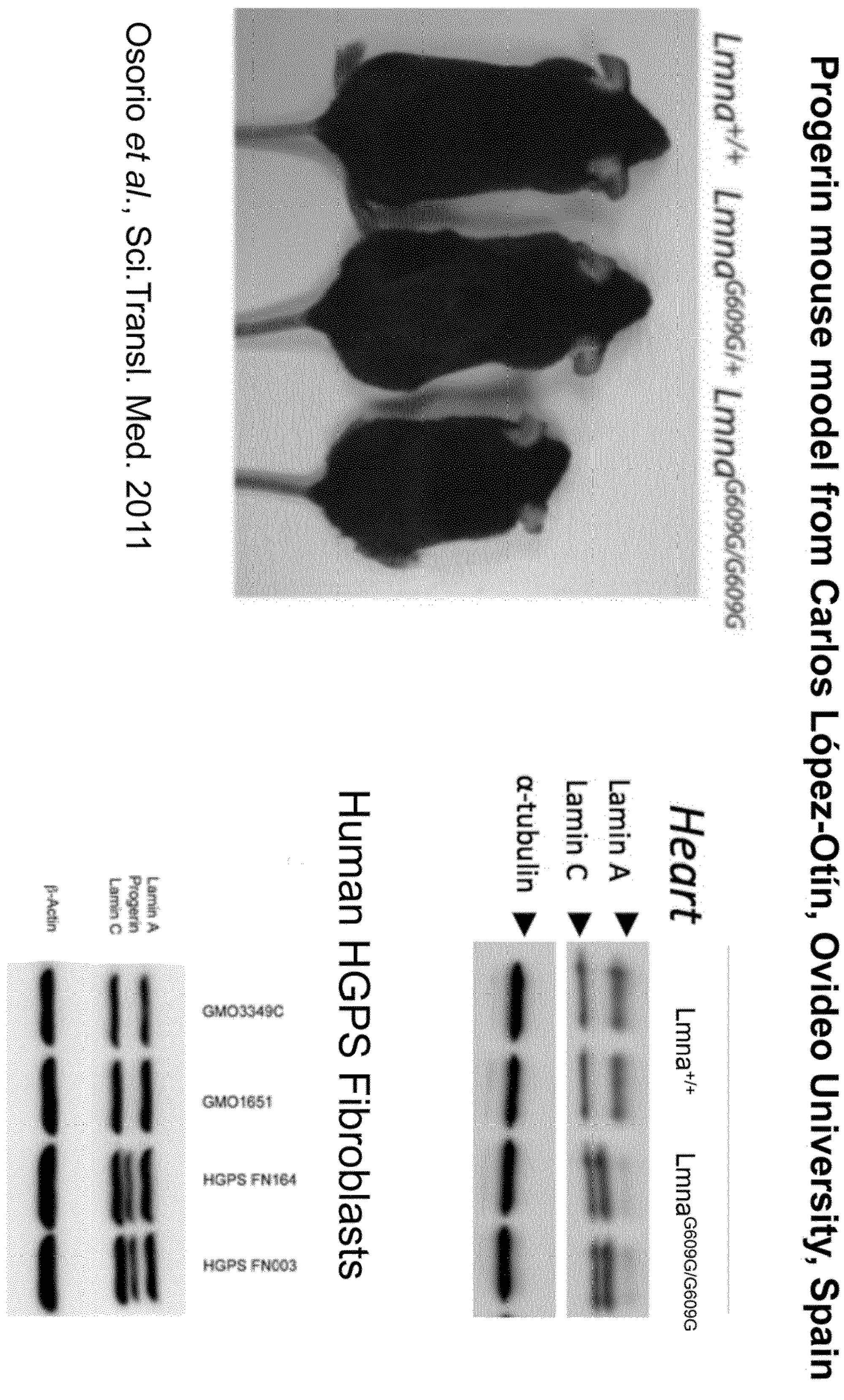
Progerin mouse model from Carlos López-Otín, Ovideo University, Spain
Lmna+/+ LmnaG609G/+ LmnaG609G/G609G
Osorio et al., Sci.Transl. Med. 2011
Heart
Lamin A
Lamin C
α-tubulin
Lmna+/+
LmnaG609G/G609G
Human HGPS Fibroblasts
Lamin A
Progerin
Lamin C
β-Actin
GMO3349C
GMO1651
HGPS FN164
HGPS FN003

FIG. 13

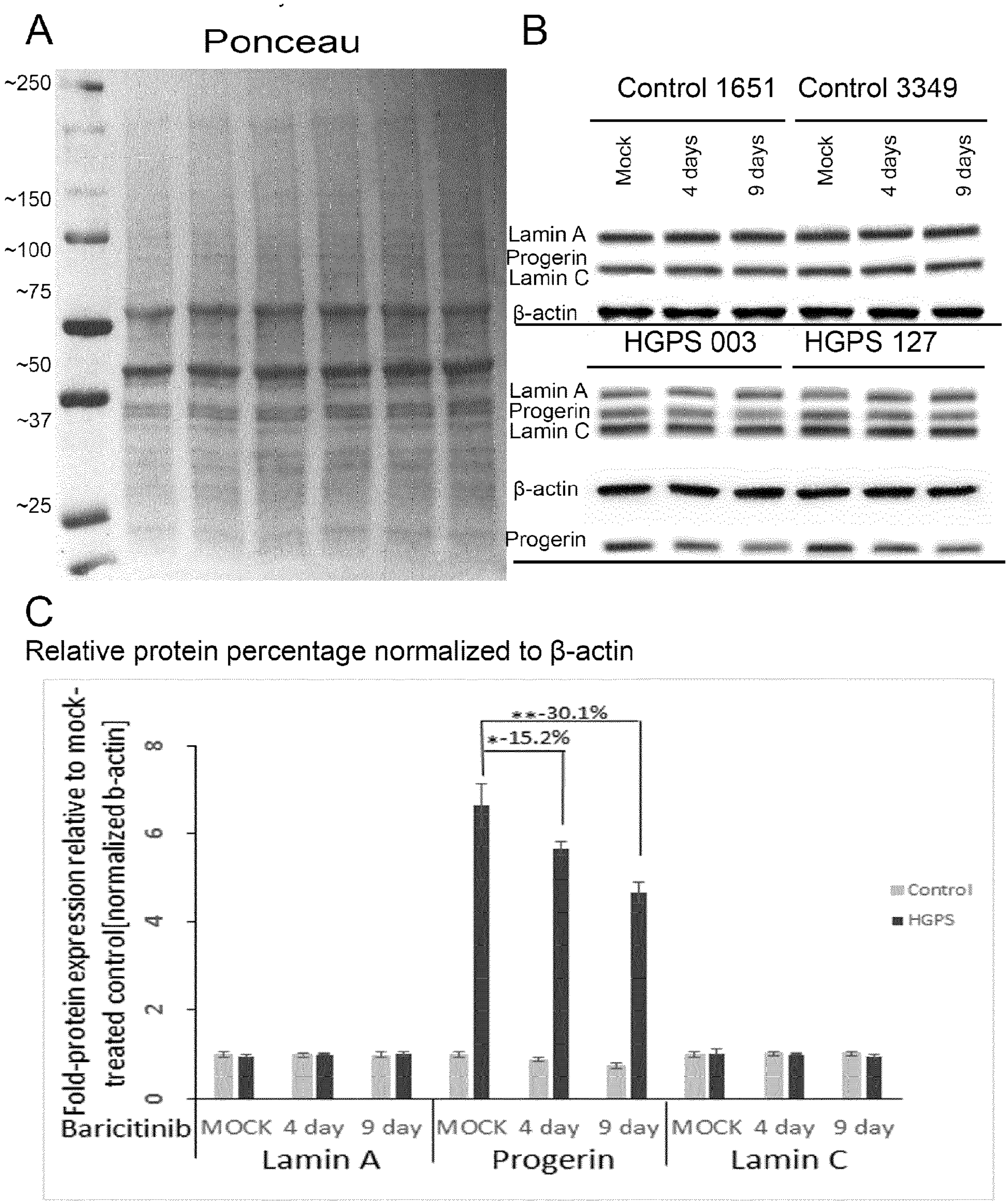
A
Ponceau
~250
~150
~100
~75
~50
~37
~25
B
Control 1651
Control 3349
Mock
4 days
9 days
Lamin A
Progerin
Lamin C
β-actin
HGPS 003
HGPS 127
C
Relative protein percentage normalized to β-actin
Fold-protein expression relative to mock-treated control[normalized b-actin]
**-30.1%
*-15.2%
Control
HGPS
Baricitinib
MOCK
4 day
9 day
Lamin A
Progerin
Lamin C

FIG. 14

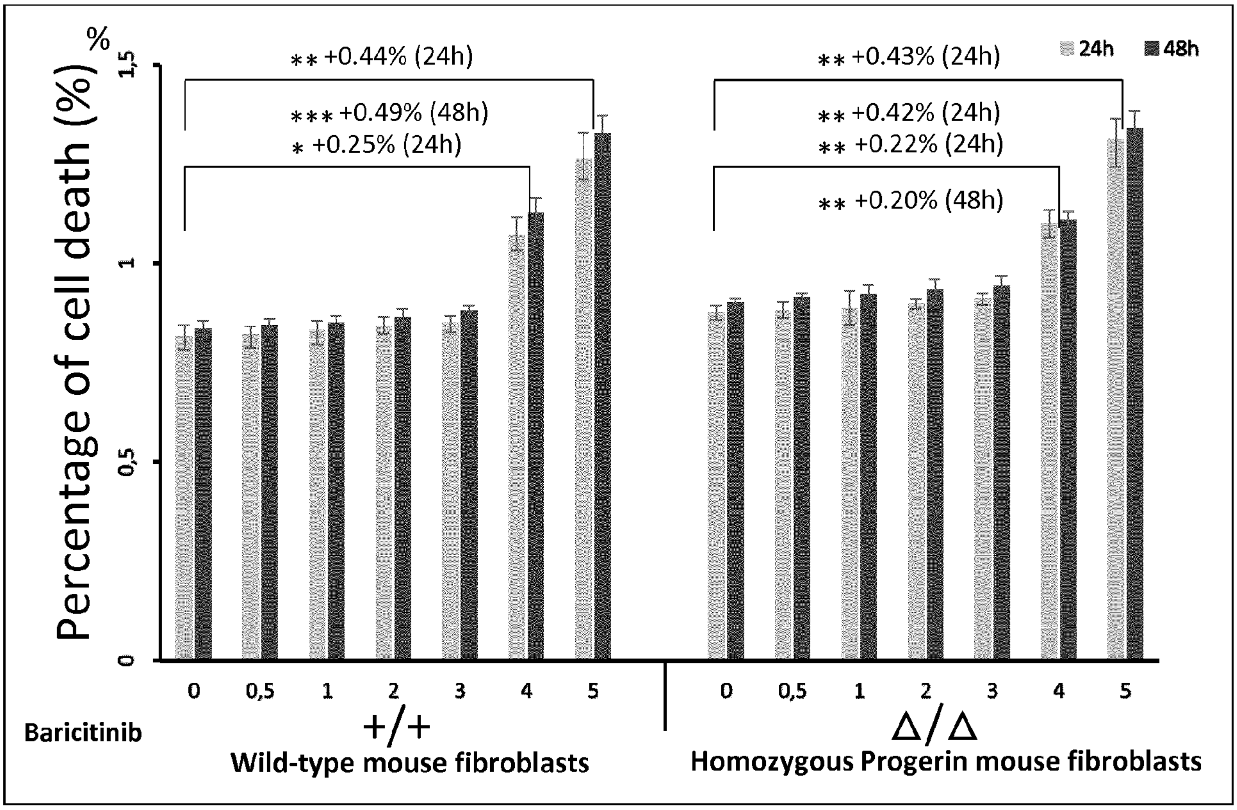

Percentage of cell death (%)
%
1,5
1
0,5
0
** +0.44% (24h)
*** +0.49% (48h)
* +0.25% (24h)
** +0.43% (24h)
** +0.42% (24h)
** +0.22% (24h)
** +0.20% (48h)
24h
48h
0
0,5
1
2
3
4
5
Baricitinib
+/+
Wild-type mouse fibroblasts
Δ/Δ
Homozygous Progerin mouse fibroblasts

FIG. 15

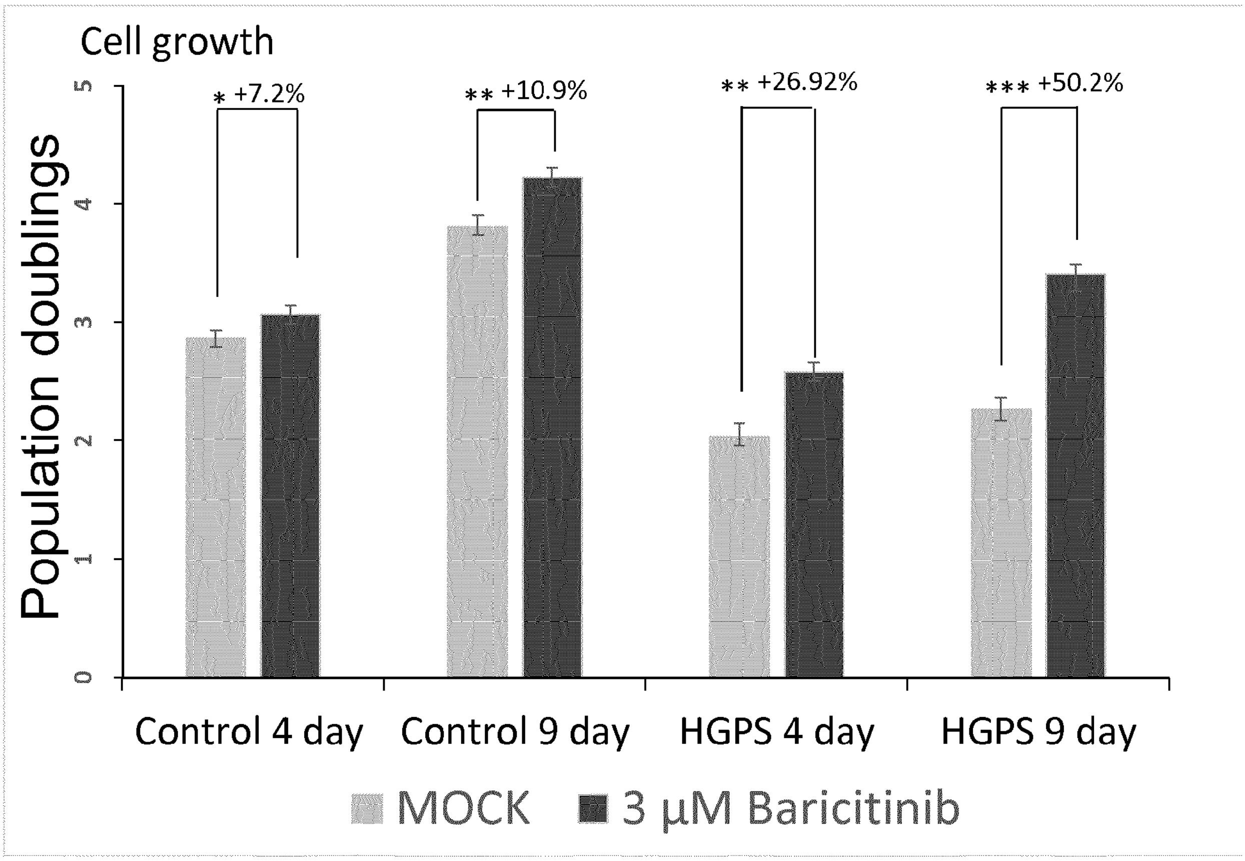
Cell growth
Population doublings
0
1
2
3
4
5
* +7.2%
** +10.9%
** +26.92%
*** +50.2%
Control 4 day
Control 9 day
HGPS 4 day
HGPS 9 day
MOCK
3 μM Baricitinib

FIG. 16

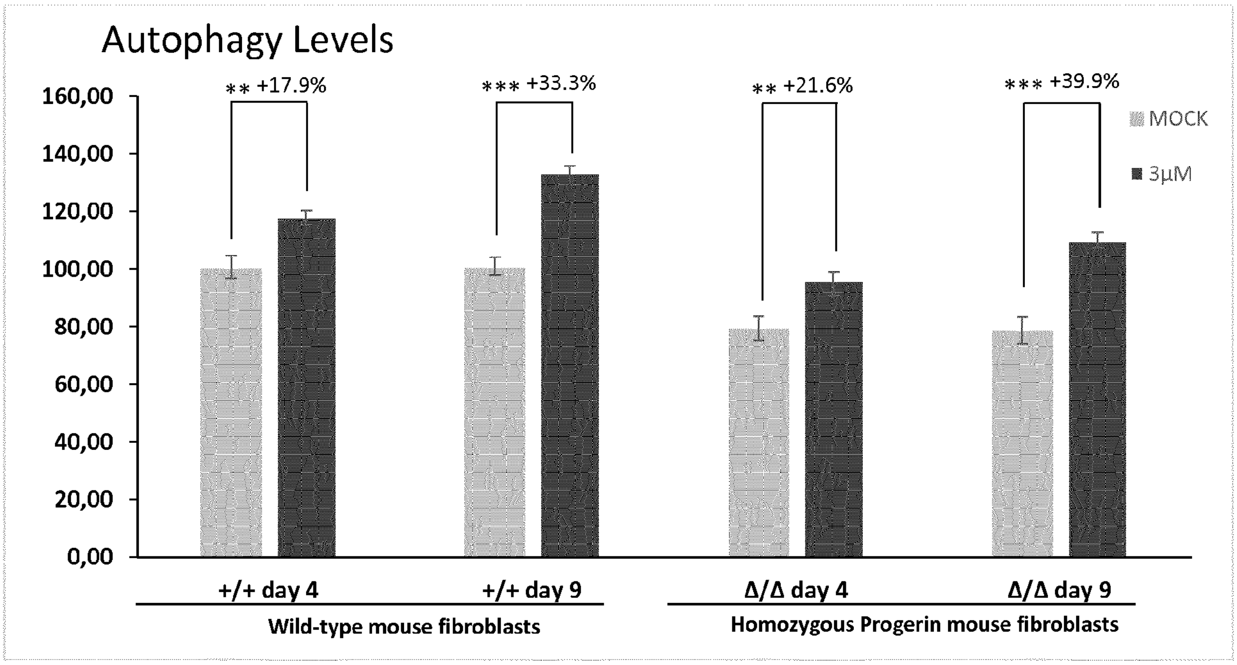
Autophagy Levels
160,00
140,00
120,00
100,00
80,00
60,00
40,00
20,00
0,00
** +17.9%
*** +33.3%
** +21.6%
*** +39.9%
MOCK
3μM
+/+ day 4
+/+ day 9
Δ/Δ day 4
Δ/Δ day 9
Wild-type mouse fibroblasts
Homozygous Progerin mouse fibroblasts

FIG. 17

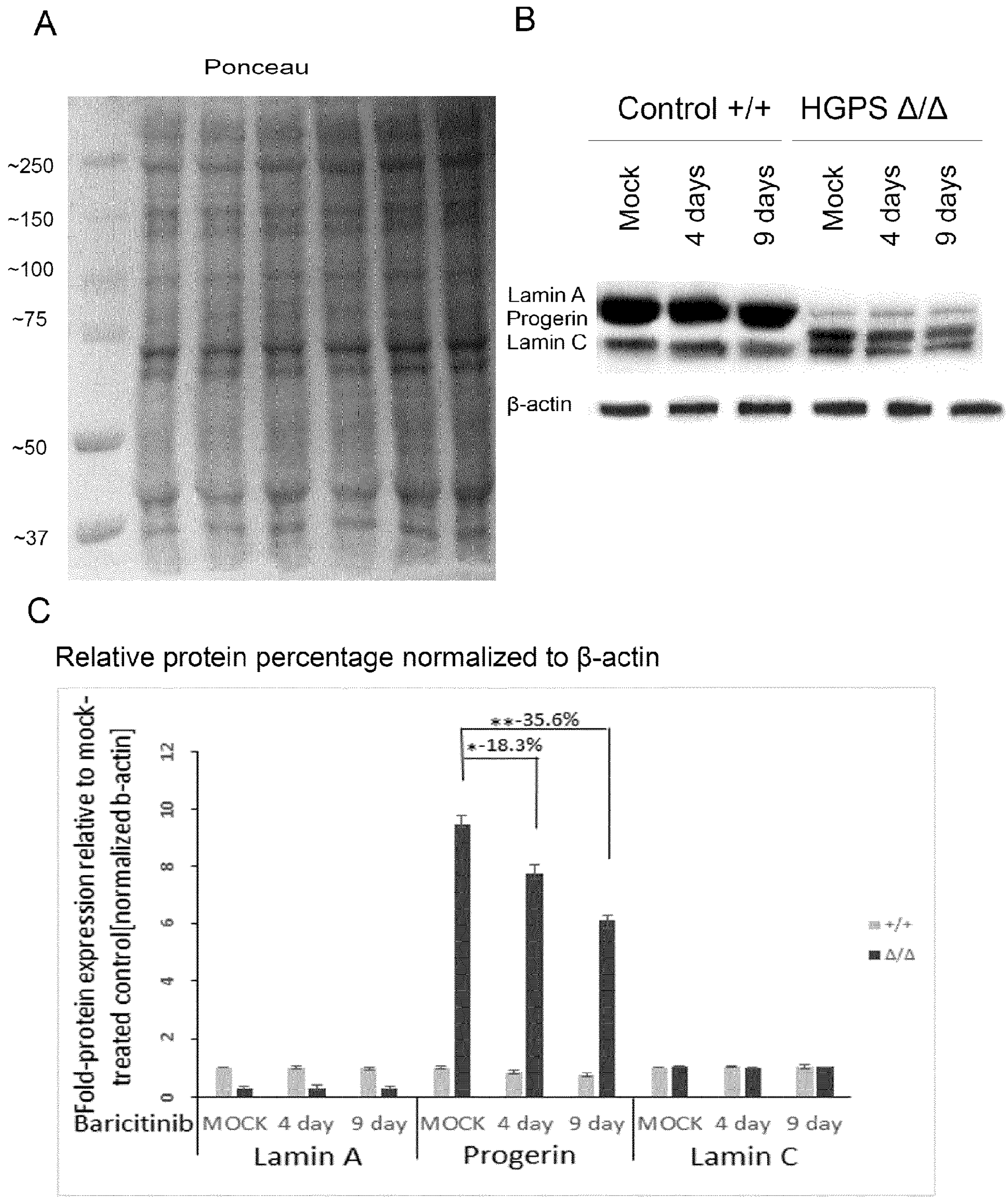
A
Ponceau
~250
~150
~100
~75
~50
~37
B
Control +/+
HGPS Δ/Δ
Mock
4 days
9 days
Mock
4 days
9 days
Lamin A
Progerin
Lamin C
β-actin
C
Relative protein percentage normalized to β-actin
Fold-protein expression relative to mock-treated control[normalized b-actin]
0
2
4
6
8
10
12
**-35.6%
*-18.3%
+/+
Δ/Δ
Baricitinib
MOCK
4 day
9 day
MOCK
4 day
9 day
MOCK
4 day
9 day
Lamin A
Progerin
Lamin C

Europäisches Patentamt
European Patent Office
Office européen des brevets

# EUROPEAN SEARCH REPORT

Application Number
EP 18 15 1570

| Category | Citation of document with indication, where appropriate, of relevant passages | Relevant to claim | CLASSIFICATION OF THE APPLICATION (IPC) |
|---|---|---|---|
| X | Goncalo Pinto De Sousa Almeida Henriques: "The connection between the JAK-STAT signaling pathway and Hutchinson-Gilford progeria syndrome", , 25 September 2017 (2017-09-25), XP055484427, Retrieved from the Internet: URL:https://fenix.tecnico.ulisboa.pt/cursos/mebiol/dissertacao/1972678479053419 [retrieved on 2018-06-14] * the whole document * & Pinto De Sousa Henriques: "Announcements . Mestrado Integrado em Engenharia Biológica: Prova pública de Mestrado de Gonçalo Pinto de Sousa Almeida Henriques", , 5 September 2017 (2017-09-05), XP055484422, Retrieved from the Internet: URL:https://fenix.tecnico.ulisboa.pt/cursos/mebiol/anuncios?p=11 [retrieved on 2018-06-14] * Prova pública de Mestrado de Gonçalo Pinto de Sousa Almeida Henriques; pages 3-4 * ----- | 1-6 | INV. A61K31/519 A61P43/00 |
| X | ANNE-LAURE EGESIPE ET AL: "Metformin decreases progerin expression and alleviates pathological defects of Hutchinson-Gilford progeria syndrome cells", NPJ AGING AND MECHANISMS OF DISEASE, vol. 2, no. 1, 10 November 2016 (2016-11-10), XP055484935, DOI: 10.1038/npjamd.2016.26 * abstract * -/-- | 1-4,6 | TECHNICAL FIELDS SEARCHED (IPC) A61K A61P |

The present search report has been drawn up for all claims

3

| Place of search | Date of completion of the search | Examiner |
|---|---|---|
| Munich | 10 July 2018 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 15 1570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & XIN-SHENG DENG ET AL: "Metformin targets Stat3 to inhibit cell growth and induce apoptosis in triple-negative breast cancers", CELL CYCLE, vol. 11, no. 2, 15 January 2012 (2012-01-15), pages 367-376, XP055484937, US ISSN: 1538-4101, DOI: 10.4161/cc.11.2.18813 * abstract * ----- | | |
| X | S BLONDEL ET AL: "Drug screening on Hutchinson Gilford progeria pluripotent stem cells reveals aminopyrimidines as new modulators of farnesylation", CELL DEATH & DISEASE, vol. 7, no. 2, 1 February 2016 (2016-02-01), pages e2105-e2105, XP055484717, DOI: 10.1038/cddis.2015.374 * abstract * * page 4; figure 2 * ----- | 1-4,6 | TECHNICAL FIELDS SEARCHED (IPC) |
| A | SOTO-GAMEZ ABEL ET AL: "Therapeutic interventions for aging: the case of cellular senescence", DRUG DISCOVERY TODAY, ELSEVIER, AMSTERDAM, NL, vol. 22, no. 5, 19 January 2017 (2017-01-19), pages 786-795, XP085018036, ISSN: 1359-6446, DOI: 10.1016/J.DRUDIS.2017.01.004 * abstract * * page 789, right-hand column, lines 1-19 * ----- -/-- | 1-6 | |

The present search report has been drawn up for all claims

| Place of search | Date of completion of the search | Examiner |
|---|---|---|
| Munich | 10 July 2018 | Garabatos-Perera, J |

3

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 18 15 1570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XU MING ET AL: "Perspective: Targeting the JAK/STAT pathway to fight age-related dysfunction", PHARMACOLOGICAL RESEARCH, ACADEMIC PRESS, LONDON, GB, vol. 111, 27 May 2016 (2016-05-27), pages 152-154, XP029725313, ISSN: 1043-6618, DOI: 10.1016/J.PHRS.2016.05.015 * the whole document * ----- | 1-6 | |
| A | YAMAOKA KUNIHIRO ED - TAWFIK DAN S ET AL: "Janus kinase inhibitors for rheumatoid arthritis", CURRENT OPINION IN CHEMICAL BIOLOGY, CURRENT BIOLOGY LTD, LONDON, GB, vol. 32, 17 March 2016 (2016-03-17), pages 29-33, XP029598624, ISSN: 1367-5931, DOI: 10.1016/J.CBPA.2016.03.006 * abstract * ----- | 1-6 | |

TECHNICAL FIELDS SEARCHED (IPC)

The present search report has been drawn up for all claims

3

| Place of search | Date of completion of the search | Examiner |
|---|---|---|
| Munich | 10 July 2018 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **OSORIO et al.** *Sci Transl. Med.,* 2001
- **ALTEN, R. ; NITSCHMANN, S.** Efficacy and safety of baricitinib in Japanese patients with rheumatoid arthritis: Subgroup analyses of four multinational phase 3 randomized trials. *Internist (Berl),* 2017, vol. 58, 1341-1344
- **CARFAGNA, M. ; CANNADY, E. ; RYAN, T. ; HERMAN, J. ; TRUEX, L. ; NARWANI, K. ; SULLIVAN, J.** Carcinogenicity assessment of baricitinib in Tg.rasH2 mice and Sprague-Dawley (Crl:CD) rats. *Regulatory in Toxicology and Pharmacology,* 2017
- **FLEISCHMANN, R. ; ALAM, J. ; ARORA, V. ; BRADLEY, J. ; SCHLICHTING, D. E. ; MURAM, D. ; SMOLEN, J. S.** Safety and efficacy of baricitinib in elderly patients with rheumatoid arthritis. *RMD Open,* 2017, vol. 3, e000546
- **GENOVESE, M. C. et al.** Baricitinib in Patients with Refractory Rheumatoid Arthritis. *New England Journal of Medecin,* 2016, vol. 374, 1243-1252
- **JABBARI, A. et al.** Reversal of Alopecia Areata Following Treatment With the JAK1/2 Inhibitor Baricitinib. *EBioMedicine,* 2015, vol. 2, 351-355
- **KARONITSCH, T. et al.** Targeted inhibition of Janus kinases abates interfon gamma-induced invasive behaviour of fibroblast-like synoviocytes. *Rheumatolog,* 2017
- **KEYSTONE, E. C. ; GENOVESE, M. C. ; SCHLICHTING, D. E. ; DE LA TORRE, I. ; BEATTIE, S. D. ; ROONEY, T. P. ; TAYLOR, P. C.** Safety and Efficacy of Baricitinib Through 128 Weeks in an Open-label, Longterm Extension Study in Patients with Rheumatoid Arthritis. *Journal of Rheumatology,* 2017, vol. 15
- **KEYSTONE, E. C. et al.** Safety and efficacy of baricitinib at 24 weeks in patients with rheumatoid arthritis who have had an inadequate response to methotrexate. *Annual Rhrumatology disease,* 2015, vol. 74, 333-340
- **KEYSTONE, E. C. et al.** Patient-reported outcomes from a phase 3 study of baricitinib versus placebo or adalimumab in rheumatoid arthritis: secondary analyses from the RA-BEAM study. *Annual Rhreumatology disease,* 2017, vol. 76, 1853-1861
- **KUBO, S. ; NAKAYAMADA, S. ; TANAKA, Y.** Baricitinib for the treatment of rheumatoid arthritis. *Expert Review in Clinical Immunology,* 2016, vol. 12, 911-919
- **LEE, Y. H. ; BAE, S. C.** Comparative efficacy and safety of baricitinib 2 mg and 4 mg in patients with active rheumatoid arthritis : A Bayesian network meta-analysis of randomized controlled trials. *Z Rheumatology,* 2017
- **MARKHAM, A.** Baricitinib: First Global Approval. *Drugs,* 2017, vol. 77, 697-704
- **MURAKAMI, K. et al.** A Jak1/2 inhibitor, baricitinib, inhibits osteoclastogenesis by suppressing RANKL expression in osteoblasts in vitro. *PLoS ONE,* 2017, vol. 12, e0181126
- **NAKAYAMADA, S. ; KUBO, S. ; IWATA, S. ; TANAKA, Y.** Recent Progress in JAK Inhibitors for the Treatment of Rheumatoid Arthritis. *BioDrug,* 2016, vol. 30, 407-419
- **NORMAN, P.** *Expert Opinion Investigative Drugs,* 2014, vol. 23, 1067-1077
- **OSORIO F. G. et al.** Splicing-Directed Therapy in a New Mouse Model of Human Accelerated Aging. *Sci Transl Med.,* October 2011, vol. 26;3 (106), 106ra107
- **SHI, J. G. ; CHEN, X. ; LEE, F. ; EMM, T. ; SCHERLE, P. A. ; LO, Y. ; PUNWANI, N. ; WILLIAMS, W. V. ; YELESWARAM, S.** The pharmacokinetics, pharmacodynamics, and safety of baricitinib, an oral JAK 1/2 inhibitor, in healthy volunteers. *Journal of Clinical Pharmacology,* 2014, vol. 54, 1354-1361
- **TANAKA, Y. ; EMOTO, K. ; CAI, Z. ; AOKI, T. ; SCHLICHTING, D. ; ROONEY, T. ; MACIAS, W.** Efficacy and Safety of Baricitinib in Japanese Patients with Active Rheumatoid Arthritis Receiving Background Methotrexate Therapy: A 12-week, Double-blind, Randomized Placebo-controlled Study. *Journal of Rheumatology,* 2016, vol. 43, 504-514
- **TAYLOR, P. C. et al.** Baricitinib versus Placebo or Adalimumab in Rheumatoid Arthritis. *New England Journal of Medecin,* 2017, vol. 376, 652-662
- **YAMAOKA, K.** Janus kinase inhibitors for rheumatoid arthritis. *Current Opinion in Chemistry and Biology,* 2016, vol. 32, 29-33

<110> Technische Universität München

<120> COMPOUNDS FOR USE IN TREATING HUTCHINSON-GILFORD PROGERIA SYNDROME

<130> ETU180101PEP

<160> 48

<170> BiSSAP 1.3.6

<210> 1
<211> 21
<212> DNA
<213> Artificial Sequence

<220>
<223> forward primer for PPAR-γ

<400> 1
gcttttcaga aatgaccatg g 21

<210> 2
<211> 23
<212> DNA
<213> Artificial Sequence

<220>
<223> reverse primer for PPAR-γ

<400> 2
tgattgcact ttggtactct tga 23

<210> 3
<211> 20
<212> DNA
<213> Artificial Sequence

<220>
<223> forward primer for CCL2

<400> 3
tagaagaatc accagcagca 20

<210> 4
<211> 19
<212> DNA
<213> Artificial Sequence

<220>

<223> reverse primer for CCL2

<400> 4
gtgcctcagt tttcccatt 19

<210> 5
<211> 20
<212> DNA
<213> Artificial Sequence

<220>
<223> forward primer for TGF-β1

<400> 5
cccacaacga aatctatgac 20

<210> 6
<211> 21
<212> DNA
<213> Artificial Sequence

<220>
<223> reverse primer for TGF-β1

<400> 6
ttgctgtatt tctggtacag c 21

<210> 7
<211> 24
<212> DNA
<213> Artificial Sequence

<220>
<223> forward primer for CXCL8

<400> 7
ggtgcataag ttctctagta gggt 24

<210> 8
<211> 20
<212> DNA
<213> Artificial Sequence

<220>
<223> reverse primer for CXCL8

<400> 8
tagaagcttg tgtgctctgc 20

<210> 9
<211> 22
<212> DNA
<213> Artificial Sequence

<220>
<223> forward primer for ICAM1

<400> 9
acagtgacca tctacagctt tc 22

<210> 10
<211> 19
<212> DNA
<213> Artificial Sequence

<220>
<223> reverse primer for ICAM1

<400> 10
ccatacagga cacgaagct 19

<210> 11
<211> 25
<212> DNA
<213> Artificial Sequence

<220>
<223> forward primer for CRP

<400> 11
aaggcaagag atctaggact tctag 25

<210> 12
<211> 20
<212> DNA
<213> Artificial Sequence

<220>
<223> reverse primer for CRP

<400> 12
tgagagaggc tggtcaagac 20

<210> 13
<211> 16
<212> DNA
<213> Artificial Sequence

<220>
<223> forward primer for C3

<400> 13
gcgcattccg attgag 16

<210> 14
<211> 18
<212> DNA
<213> Artificial Sequence

<220>
<223> reverse primer for C3

<400> 14
tcactgcctg agtgcaag 18

<210> 15
<211> 18
<212> DNA
<213> Artificial Sequence

<220>
<223> forward primer for TRAF1

<400> 15
cagagggtgg tggagctt 18

<210> 16
<211> 20
<212> DNA
<213> Artificial Sequence

<220>
<223> reverse primer for TRAF1

<400> 16
agtgtagaag gctggggaga 20

<210> 17
<211> 17
<212> DNA
<213> Artificial Sequence

<220>
<223> forward primer for IL-18

<400> 17
ttgtcgcaga tggctct 17

<210> 18
<211> 25
<212> DNA
<213> Artificial Sequence

<220>
<223> reverse primer for IL-18

<400> 18
ttaccatcat cttcagctat aaagt 25

<210> 19
<211> 24
<212> DNA
<213> Artificial Sequence

<220>
<223> forward primer for IGF1

<400> 19
gattacacct acagtgaaga tgca 24

<210> 20
<211> 20
<212> DNA
<213> Artificial Sequence

<220>
<223> reverse primer for IGF1

<400> 20
cctgtgggct tgttgaaata 20

<210> 21
<211> 21
<212> DNA
<213> Artificial Sequence

<220>
<223> forward primer for IL-6

<400> 21
aaggagagtc acaggtgagc t 21

<210> 22
<211> 22
<212> DNA
<213> Artificial Sequence

<220>
<223> reverse primer for IL-6

<400> 22
caagtctcct cattgaatcc ag 22

<210> 23
<211> 17
<212> DNA
<213> Artificial Sequence

<220>
<223> forward primer for TNF-α

<400> 23
gagggaagag ttcccca 17

<210> 24
<211> 20
<212> DNA
<213> Artificial Sequence

<220>
<223> reverse primer for TNF-α

<400> 24
cttgagggtt tgctacaaca 20

<210> 25
<211> 18
<212> DNA
<213> Artificial Sequence

<220>
<223> forward primer for FAS

<400> 25
tcctccaggt gaaaggaa 18

<210> 26
<211> 21
<212> DNA
<213> Artificial Sequence

<220>
<223> reverse primer for FAS

<400> 26
acttctaagc catgtccttc a 21

<210> 27
<211> 20
<212> DNA
<213> Artificial Sequence

<220>
<223> forward primer for STAT1

<400> 27
ttcagagctc gtttgtggtg 20

<210> 28
<211> 20
<212> DNA
<213> Artificial Sequence

<220>
<223> reverse primer for STAT1

<400> 28
agaggtcgtc tcgaggtcaa 20

<210> 29
<211> 20
<212> DNA
<213> Artificial Sequence

<220>
<223> forward primer for STAT2

<400> 29
ccaaggctac catgctattc 20

<210> 30
<211> 20
<212> DNA
<213> Artificial Sequence

<220>
<223> reverse primer for STAT2

<400> 30
gctggtcttt cagttggctg 20

<210> 31
<211> 20
<212> DNA
<213> Artificial Sequence

<220>
<223> forward primer for STAT3

<400> 31
caggagggca gtttgagtcc 20

<210> 32
<211> 22
<212> DNA
<213> Artificial Sequence

<220>
<223> reverse primer for STAT3

<400> 32
caaagatagc agaagtagga ga 22

<210> 33
<211> 23
<212> DNA
<213> Artificial Sequence

<220>
<223> forward primer for STAT4

<400> 33
cctgacattc ccaaagacaa agc 23

<210> 34
<211> 22
<212> DNA
<213> Artificial Sequence

<220>
<223> reverse primer for STAT4

<400> 34
tctctcaaca ccgcatacac ac 22

<210> 35
<211> 22
<212> DNA
<213> Artificial Sequence

<220>
<223> forward primer for STAT5A

<400> 35

aagccaacaa ttgcagctct cc 22

<210> 36
<211> 22
<212> DNA
<213> Artificial Sequence

<220>
<223> reverse primer for STAT5A

<400> 36
ggcaaactga gcttggatcc tc 22

<210> 37
<211> 25
<212> DNA
<213> Artificial Sequence

<220>
<223> forward primer for STAT5B

<400> 37
caagcatggg actcagtaga tcttg 25

<210> 38
<211> 23
<212> DNA
<213> Artificial Sequence

<220>
<223> reverse primer for STAT5B

<400> 38
cagctggaga gctaccattg ttg 23

<210> 39
<211> 27
<212> DNA
<213> Artificial Sequence

<220>
<223> forward primer for STAT6

<400> 39
atcagcaccc ttgagagcat atatcag 27

<210> 40
<211> 21
<212> DNA

<213> Artificial Sequence

<220>
<223> reverse primer for STAT6

<400> 40
ctgtgcagag acacttggcc a 21

<210> 41
<211> 19
<212> DNA
<213> Artificial Sequence

<220>
<223> forward primer for JAK1

<400> 41
aatggcgaga tccccttga 19

<210> 42
<211> 23
<212> DNA
<213> Artificial Sequence

<220>
<223> reverse primer for JAK1

<400> 42
gcaccggctt tcatagaatc tct 23

<210> 43
<211> 20
<212> DNA
<213> Artificial Sequence

<220>
<223> forward primer for JAK2

<400> 43
tgattttgtg cacggatgga 20

<210> 44
<211> 22
<212> DNA
<213> Artificial Sequence

<220>
<223> reverse primer for JAK2

<400> 44
actgccatcc caagacattc tt 22

<210> 45
<211> 19
<212> DNA
<213> Artificial Sequence

<220>
<223> forward primer for JAK3

<400> 45
gcctggagtg gcatgagaa 19

<210> 46
<211> 20
<212> DNA
<213> Artificial Sequence

<220>
<223> reverse primer for JAK3

<400> 46
ccccggtaaa tcttggtgaa 20

<210> 47
<211> 20
<212> DNA
<213> Artificial Sequence

<220>
<223> forward primer for TYK2

<400> 47
gggaccgtgg gcaggagcta 20

<210> 48
<211> 20
<212> DNA
<213> Artificial Sequence

<220>
<223> reverse primer for TYK2

<400> 48
gtgcgtgtgg gagacctggc 20